# EUROPEAN PATENT SPECIFICATION

(11) **EP 2 510 008 B1**
(45) Date of publication and mention of the grant of the patent: **21.10.2015**
(21) Application number: 10787779.7
(22) Date of filing: 09.12.2010
(51) Int. Cl.: C07K 16/28, C07K 16/46

(54) **Trispecific therapeutics against acute myeloid leukaemia**
Trispezifische Therapeutika zur Behandlung akuter myeloischer Leukämie
Thérapie trispécifique contre la leucémie myéloïde aiguë

(30) Priority: 09.12.2009 EP 09015260
(43) Date of publication of application: 17.10.2012
(73) Proprietor: Friedrich-Alexander-Universität Erlangen-Nürnberg, 91054 Erlangen (DE)
(72) Inventor: FEY, Georg H., 91077 Neunkirchen a. Br. (DE); STEIN, Christoph, 52080 Aachen (DE); KELLNER, Christian, 24143 Kiel (DE); KÜGLER, Markus, 81476 München (DE)
(74) Representative: Vossius & Partner Patentanwälte Rechtsanwälte mbB
(86) International application number: PCT/EP2010/069271
(87) International publication number: WO 2011/070109

(56) References cited:
- WO-A1-2009/007124
- WO-A1-2009/070844
- DU X ET AL: "New immunotoxins targeting CD123, a stem cell antigen on acute myeloid leukemia cells", JOURNAL OF IMMUNOTHERAPY 200709 US, vol. 30, no. 6, September 2007 (2007-09), pages 607-613, XP008116347, ISSN: 1524-9557
- SILLA L M R ET AL: "POTENTIATION OF LYSIS OF LEUKAEMIA CELLS BY A BISPECIFIC ANTIBODY TO CD33 AND CD16 (FCGAMMARIII) EXPRESSED BY HUMAN NATURAL KILLER (NK) CELLS", BRITISH JOURNAL OF HAEMATOLOGY, WILEY-BLACKWELL PUBLISHING LTD, GB, vol. 89, 1 January 1995 (1995-01-01), pages 712-718, XP002048530, ISSN: 0007-1048
- STEIN CHRISTOPH ET AL: "Novel conjugates of single-chain Fv antibody fragments specific for stem cell antigen CD123 mediate potent death of acute myeloid leukaemia cells.", BRITISH JOURNAL OF HAEMATOLOGY MAR 2010 LNKD- PUBMED:20064159, vol. 148, no. 6, March 2010 (2010-03), pages 879-889, XP002618076, ISSN: 1365-2141
- KÜGLER MARKUS ET AL: "A recombinant trispecific single-chain Fv derivative directed against CD123 and CD33 mediates effective elimination of acute myeloid leukaemia cells by dual targeting.", BRITISH JOURNAL OF HAEMATOLOGY SEP 2010 LNKD- PUBMED:20636437, vol. 150, no. 5, September 2010 (2010-09), pages 574-586, XP002618077, ISSN: 1365-2141
- FLORIAN STEFAN ET AL: "Detection of molecular targets on the surface of CD34+/CD38-- stem cells in various myeloid malignancies", LEUKEMIA AND LYMPHOMA, INFORMA HEALTHCARE, US, vol. 47, no. 2, 1 February 2006 (2006-02-01), pages 207-222, XP009127675, ISSN: 1042-8194, DOI: 10.1080/10428190500272507
- VARDIMAN JAMES W ET AL: "The 2008 revision of the World Health Organization (WHO) classification of myeloid neoplasms and acute leukemia: rationale and important changes.", BLOOD 30 JUL 2009, vol. 114, no. 5, 30 July 2009 (2009-07-30) , pages 937-951, ISSN: 1528-0020
- JILANI IMAN ET AL: "Differences in CD33 intensity between various myeloid neoplasms.", AMERICAN JOURNAL OF CLINICAL PATHOLOGY OCT 2002, vol. 118, no. 4, October 2002 (2002-10), pages 560-566, ISSN: 0002-9173

## Description

The present invention relates to a molecule having binding specificities for (a) CD123; (b) CD16 and (c) CD33, as characterised in the appended claims. The present invention further relates to the molecule of the invention, wherein the molecule comprises a first immunoglobulin domain comprising a V_{L} domain linked to a V_{H} domain, wherein the immunoglobulin domain specifically binds to CD123; a second immunoglobulin domain comprising a V_{L} domain linked to a V_{H} domain, wherein the immunoglobulin domain specifically binds to CD16; and a third immunoglobulin domain comprising a V_{L} domain linked to a V_{H} domain, wherein the immunoglobulin domain specifically binds to CD33. The present invention furthermore relates to a nucleic acid molecule encoding the molecule of the invention. In addition, the present invention relates to diagnostic and pharmaceutical compositions and the use of the molecule or the nucleic acid molecule of the invention in the treatment of acute myeloid leukaemia and/or myelodysplastic syndrome.

In this specification, a number of documents including patent applications and manufacturer's manuals is cited.

Acute myeloid leukaemia (AML) is the second most common acute leukaemia with approximately 13,300 new cases per year in the United States and 8,800 annual deaths (Jemal *et al*, 2008). The commonly applied therapy of leukaemic diseases includes irradiation and/or chemotherapy. Furthermore, under certain circumstances, the additional possibility of bone marrow transplantation is regarded suitable. However, these therapies are relatively toxic to the patient and very often do not lead to a complete cure from the disease. Thus, although a complete remission can be achieved for 65-80% of all patients receiving chemotherapy (Cros *et al*, 2004, Kern and Estey 2006), most of these patients relapse (Cros *et al*, 2004). In the remission phase after an initial course of conventional chemotherapy the blast counts are reduced in the patient's bone marrow to approximately 5% or less of total bone marrow leukocytes, and the cells that survived the chemotherapy are referred to as "minimal residual disease" (MRD) cells. These cells are enriched in AML leukaemia stem cells (AML-LSCs), which are particularly resistant to chemotherapy, and they constitute a particularly dangerous reservoir of cells capable of re-expanding and causing a relapse. Leukaemia stem cells have been particularly well characterized for acute myeloid leukaemia (Lapidot *et al*, 1994, Bonnet and Dick 1997, Hope *et al*, 2004). AML-LSCs express a characteristic set of cell-surface antigens including among others CD123, C-type lectin-like molecule-1 (CLL-1), CD44, CD33 and for a subset of AML cases CD96 (Jordan *et al*, 2000, Bakker *et al*, 2004, Jin *et al*, 2006, Hauswirth *et al*, 2007, Hosen *et al*, 2007, Misaghian *et al*, 2009). These distinguished cells exhibit unique biological properties, including infrequent entrance into the cell cycle, self-renewal potential, and an enhanced resistance to chemotherapeutic agents and DNA damage. They are therefore likely to contribute significantly to the population of minimal residual disease (MRD) cells and are responsible for relapse in conventionally treated AML-patients (Ravandi and Estrov 2006). The 4-year disease-free survival of patients that are younger than 60 years and who receive a standard chemotherapy only reaches approximately 40%. Furthermore, patients older than 60 years have a poor prognosis with only 10% to 15% of 4-year disease-free survival (Mayer *et al*, 1994, Gardin *et al*, 2007)..This high relapse rate for AML patients and the poor prognosis for older patients highlight the urgent need for novel therapeutics preferentially targeting the AML-LSCs.

An FDA approved drug for the treatment of AML is Gemtuzumab Ozogamicin (GO, Mylotarg™, Wyeth, Madison, NJ, USA), which is a CD33-specific drug. The use of this agent is restricted to patients older than 60 years in first relapse and to patients resistant against standard chemotherapy (Bross *et al*, 2001, Sievers 2001). GO consists of a humanized anti-CD33 IgG-antibody, chemically coupled to the cytotoxic agent calicheamicin (Hamann *et al*, 2002). In a phase II clinical trial, 30% of relapsed AML patients responded to GO (Sievers 2001, Larson *et al*, 2002). However, side effects were found to include hepatic veno-occlusive disease, pulmonary toxicity and severe hypersensitivity reactions. Furthermore, *in vitro* studies revealed antigen-independent cytotoxicities towards CD33 negative cell lines (Bross *et al*, 2001, Jedema *et al*, 2004, Schwemmlein *et al*, 2006).

In the past, different approaches have been used to develop unconjugated monoclonal antibodies with improved antitumor activity. One of these approaches was the generation of bispecific antibodies by a variety of methods. In general, these molecules consist of one binding site for a target antigen on tumor cells and a second binding side for an activating trigger molecule on an effector cell, such as CD3 on T-cells, CD16 (FcγRIII) on natural killer (NK) cells, monocytes and macrophages, CD89 (FcαRI) and CD64 (FcγRI) on neutrophils and monocytes/macrophages, and DEC-205 on dendritic cells (Peipp and Valerius 2002, Wang *et al*, 2005). Apart from the specific recruitment of the preferred effector cell population, bispecific antibodies avoid competition with endogenous immunoglobulin G (IgG) when the selected binding site for the trigger molecule on the effector cell does not overlap with Fc-binding epitopes. In addition, the use of single-chain Fv fragments instead of full-length immunoglobulin prevents the molecules from binding to Fc-receptors on non-cytotoxic cells, such as FcγRII on platelets and B-cells, to Fc-receptors that do not activate cytotoxic cells, including FcγRIIIb on polymorphonuclear leukocytes (PMN), and to inhibitory Fc-receptors, such as FcγRIIb on monocytes/macrophages (Daeron 1997).
A number of bispecific single-chain Fv (bsscFv) fusion proteins are known in the art with cytolytic activity for leukaemia-derived cells, including Bispecific diabodies directed against CD19 and CD16 (Kipriyanov *et al*, 2002), tandem bsscFvs directed against CD19 and CD16 (Bruenke *et al*, 2005) and Human Leukocyte Antigen class II and CD16 (Bruenke *et al*, 2004). These proteins recruit CD16 positive NK-cells and monocytes/macrophages as effector cells, both important effector cell populations *in vivo* (Uchida *et al*, 2004). Another group of molecules, including an MCSP (Melanoma-associated Chondroitin Sulfate Proteoglycan) x CD28 (Otz *et al*, 2009) as well as a CD19 x CD3 (Bargou *et al*, 2008) construct, recruit cytolytic T-cells as effectors. The CD19 x CD3 molecule is the first recombinant Bispecific single chain Fv to have produced promising results in clinical phase I and ongoing phase II studies (Nagorsen *et al*, 2009).

Although bsscFvs and some of the alternative formats offer distinct advantages, they still require further improvement. The critical parameters determining the therapeutic efficacy of bispecific proteins are affinity, valence, stability, and size. Many scFv fragments display lower affinity than the corresponding parental monoclonal antibodies (mAbs) owing to losses incurred during the conversion to the recombinant scFv format (Huston et *al*, 1988). Also, scFvs often show characteristically reduced thermal stability, owing to a tendency to unfold and aggregate, which is a concern for drug approval and clinical applications (Willuda *et al*, 1999). In addition, bsscFvs in their simplest format, consisting of two scFvs connected by a flexible linker of approximately 10 to 20 amino acids, have a relative molecular mass (Mr) of only about 50 to 60 kDa, and proteins with Mr ≤65 kDa are rapidly cleared from the bloodstream by the kidneys (Kipriyanov *et al*, 1999, Huhalov and Chester 2004). Finally, as bsscFvs are devoid of an Fc portion, they also lack the interaction domain for the neonatal FcR, which is embedded in the Fc domain. The neonatal FcR facilitates recirculation of intact IgG (Raghavan *et al*, 1994). Rapid clearance from the blood results in poor retention at the tumor site (Hu *et al*, 1996).

To overcome these problems, a number of improvements have been made. One was the addition of an artificial intramolecular disulphide bond between the V_{H} chains and V_{L} chains of an scFv by *in vitro* mutagenesis, termed "disulphide stabilization" (Reiter *et al*, 1994, Bruenke *et al*, 2005). The extra bond prevents unfolding.and denaturation of the scFv and results in a significant gain in stability (Reiter *et al*, 1994). The size of the bispecific proteins has been increased by various modifications, including PEGylation (Kubetzko *et al*, 2006); the addition of further protein domains, such as human serum albumin (Huhalov and Chester 2004, Muller *et al*, 2007); or the addition of an extra scFv component (Schoonjans *et al*, 2000). These modifications resulted in improved plasma and body retention times *in vivo* (Kontermann 2005). A further improvement was achieved through *in vitro* affinity maturation of the scFv components of a bispecific protein (McCall *et al*, 2001). An increase in affinity can increase the cytotoxic potential of intact antibodies and bispecific antibodies *in vitro* (McCall *et al*, 2001, Tang *et al*, 2007) and favors the specific tumor retention of scFvs *in vivo* (Adams *et al*, 1998). Finally, an important improvement in the cytolytic potential of bispecific proteins has been achieved by increasing their valence by including a second or further binding sites for target antigens on the tumor cell (Shahied *et al*, 2004). A number of different formats of antibody-derived proteins with increased valence and increased mass have been described, including antibody-scFv fusions (Coloma and Morrison 1997), minibodies (Hu *et al*, 1996, Shahied *et al*, 2004), Fab-scFv fusions (Schoonjans *et al*, 2000), multimeric scFvs such as triabodies and tetrabodies (Todorovska *et al*, 2001), tandem scFv diabodies (Kipriyanov *et al*, 1999) and di-diabodies (Lu *et al*, 2003, Muller and Kontermann 2007).

WO 2009/007124 describes an additional format to overcome the limitations of bsscFv by providing a novel single-chain construct. This molecule consists of three scFvs covalently linked in tandem, two with specificity for target antigens on the tumor cells and one for the trigger molecule on the effector cells. In one example this molecule is directed against one molecule of CD16 and two molecules of CD33. This format, termed single-chain Fv triplebody (sctb) differs from the multimeric scFvs, triabodies, and tetrabodies, which are multichain constructs, by being a single-chain polypeptide.

Vardiman et al. 2009 (Blood 114:937) provides an overview of the 2008 revision of the World Health Organisation (WHO) classification of myeloid neoplasms and acute leukemia. Jilani et al. 2002 (Am J Clin Pathol 118:560) provide a comparative study regarding the expression of CD33 in various myeloid diseases, and observe that in the bone marrow the mean cell surface density of CD33 among all CD33-positive cells averaged over all the patients of the panel was in the range of 10 000 copies per cell for AML, and in the range of 6 000 to 7 000 copies per cell for MDS. In Florian et al. 2006 (Leukemia & Lymphoma 47(2):207) it was shown that all MDS patients co-expressed CD33 and CD123 both on their blasts and in the subcompartment of CD34+CD38- cells, which encompasses the MDS cancer stem cells. Despite the above described advances in the development of therapeutically relevant antibody constructs, no satisfying therapeutic is at present available for the treatment of acute myeloid leukaemia as well as myelodysplastic syndrome.

This need is addressed by the provision of the embodiments characterized in the claims.

Accordingly, the present invention relates in a first embodiment to a molecule having binding specificities for (a) CD123; (b) CD16 and (c) CD33, as characterised in the appended claims.

In accordance with the invention, it is to be understood that the binding specificities are conferred by different portions of the molecule, which preferably are of modular form. In a preferred embodiment, those portions of the molecule having binding specificities for CD123, CD16 and CD33 are of proteinaceous nature, e.g. polypeptides or amino acid sequences within such polypeptides. These amino acid sequences may be consecutive amino acid sequences within the polypeptide. Alternatively, amino acids or stretches of amino acids from different portions of the polypeptide may confer binding specificity to the molecule. More preferably, the entire molecule of the invention is a polypeptide.

The term "polypeptide" as used herein describes linear molecular chains of amino acids, including single chain proteins or their fragments, containing more than 30 amino acids. Accordingly, the term "peptide" as used in the present invention describes linear chains of amino acids containing up to 30 amino acids. The term "(poly)peptide" as used in accordance with the present invention refers to a group of molecules which comprises the group of peptides, consisting of up to 30 amino acids, as well as the group of polypeptides, consisting of more than 30 amino acids.
Furthermore, peptidomimetics of such proteins/polypeptides are also contemplated, wherein amino acid(s) and/or peptide bond(s) have been replaced by functional analogues. Such functional analogues include all known amino acids other than the 20 gene-encoded amino acids, such as selenocysteine. The terms "polypeptide" and "protein", which are used interchangeably, also refer to naturally modified polypeptides/proteins where the modification is effected e.g. by glycosylation, acetylation, phosphorylation and similar modifications which are well known in the art.

The term "having binding specificities" in accordance with the present invention means that parts of the molecule of the invention (i.e. the binding portions thereof) bind to their respective targets, i.e. the antigens CD123, CD16 and CD33. It is particularly preferred that they bind their respective targets specifically. The terms "specifically binds" and "specifically binding" (having the same meaning as "specifically interacting") as used in accordance with the present invention mean that these binding portions do not or essentially do not cross-react with an epitope with a structure similar to that of the target antigen. Cross-reactivity of a panel of molecules under investigation may be tested, for example, by assessing binding of said panel of molecules under conventional conditions to the epitope of interest as well as to a number of more or less (structurally and/or functionally) closely related epitopes. Only those molecules that bind to the epitope of interest in its relevant context (e.g. a specific motif in the structure of a protein) but do not or do not essentially bind to any of the other epitopes are considered specific for the epitope of interest and thus to be molecules in accordance with this invention. Corresponding methods are described e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. One exemplary molecules in accordance with the present invention is a molecule that binds CD16 and specifically binds CD123 and CD33. Thus, this preferred molecule binds CD123 and CD33 without any cross-reactivity for other target antigens while the molecule may comprise a CD16-binding portion that is capable of binding other target antigens. A more preferred molecule in accordance with the present invention is a molecule that specifically binds all of CD16, CD123 and CD33 and does not cross-react with any other antigen. Methods for achieving specific binding also for CD16 are well known in the art and are, for example, described in the examples below.
The binding portions of the molecule of the invention having binding specificities for the antigens listed in (a) to (c) of the molecule of the invention may be arranged in any order within said molecule, such as for example (a)-(b)-(c), (b)-(c)-(a), (c)-(a)-(b), (b)-(a)-(c) etc. More preferably, the binding portions of (a) to (c) are arranged in the recited order (e.g. for proteinaceous sequences they would be arranged in the order (a)-(b)-(c) either in the N-terminus to C-terminus direction or, alternatively, in the C-terminus to N-terminus direction).

As detailed above, in the remission phase after an initial course of conventional chemotherapy the blast counts are reduced in the patient's bone marrow to approximately 5% or less of total bone marrow leukocytes. The cells that survived the chemotherapy, the "minimal residual disease" cells, are enriched in AML leukaemia stem cells (AML-LSCs). The ineffective elimination of these critical tumor progenitor cells is considered to be the cause for the high number of relapses seen in AML patients after chemotherapy. The discovery of these tumor-repopulating cells therefore has important clinical implications, because their specific elimination promises to offer a most favorable opportunity to improve AML-treatment. AML stem cells express a characteristic set of cell-surface antigens including among others CD123, C-type lectin-like molecule-1 (CLL-1), CD44, CD33 and for a subset of AML cases CD96 (Jordan *et al*, 2000, Bakker *et al*, 2004, Jin *et al*, 2006, Hauswirth *et al*, 2007, Hosen *et al*, 2007, Misaghian *et al*, 2009). About 60% of the AML-LSCs express CD33 and about 70% CD123 (Taussig *et al*, 2005, Hauswirth *et al*, 2007).

CD123, the alpha subunit of the interleukin-3 receptor (IL-3Ra), is expressed on a variety of hematopoietic cells, including mostly myeloid cells, but also on a subpopulation of B lymphocytes. It is not expressed on peripheral T-cells, natural killer cells (NK-cells), platelets and red blood cells (Moretti *et al*, 2001). CD123 is widely expressed in hematopoietic malignancies and has also been detected on AML-LSCs (Jordan *et al*, 2000, Munoz *et al*, 2001, Testa *et al*, 2004, Taussig *et al*, 2005, Jin *et al*, 2009), while it is present only in lower density on normal hematopoietic stem cells (Huang *et al*, 1999, Jin *et al*, 2009). Thus, CD123 shows a 4-fold increased expression on AML-LSCs in comparison to normal HSCs which makes it a particular interesting target for therapeutics (Jin *et al*, 2009).

CD33 is a 67 kDa cell surface glycoprotein present on blasts of more than 80% of AML patients and on normal myeloid cells. The antigen is not found on tissues outside the hematopoietic system but it is discussed to be expressed on a subset of normal hematopoietic stem cells (HSC) (Dinndorf *et al*, 1986, Grossbard *et al*, 1992, Legrand *et al*, 2000, Sievers 2001, Taussig *et al*, 2005).

CD16 is the low affinity receptor for IgG (FcγRIII), which is constitutively expressed as a transmembrane isoform on the surface of NK cells and macrophages (CD16a), and as a glycosyl phosphatidyl inositol (GPI)-anchored molecule on the surface of neutrophils (CD16b) (Ravetch and Kinet, 1991; van deWinkel and Anderson, 1991). For intracellular signalling, CD16a requires association with the FcRy chain containing the immunoreceptor tyrosine-based activation motif (ITAM), which triggers downstream signalling. Studies with conventionally coupled bi-specific antibodies redirecting NK cells via CD16 demonstrated potent cytolysis of cultured malignant cells and in animal models (de Palazzo et al, 1992; Hombach et al, 1993; Kipriyanov et al, 2002). Therefore, clinical trials with CD16-directed bi-specific antibodies were initiated (Weiner et al, 1995; Hartmann et al, 1997). However, immunogenicity of hybrid-hybridoma antibodies, as well as undesired side effects caused by the presence of Fc-domains, and difficulties in producing sufficient amounts of clinical-grade material limited these clinical trials.

The molecule of the present invention enables the preferential targeting of AML-LSCs by using a dual targeting approach with CD123 as the main target and CD33 serving as an additional validated target antigen for the treatment of AML. Specific binding of the molecule of the invention to CD16 furthermore allows the recruitment of effector cells, which are able to eliminate the AML leukaemia stem cells targeted by the molecule of the invention. The therapeutic effect achieved is both the result of direct elimination of the tumor cell by antibody dependant cellular cytotoxicity (ADCC, also referred to as "redirected lysis") as well as the generation of apoptotic fragments of AML cells resulting from their lysis. These fragments are subsequently taken up by phagocytes and are processed and presented again by CD4⁺ and CD6⁺ T-lymphocytes. Both mechanisms help to build up a secondary titer of humoral antitumor antibodies and a cellular anti-tumor response by CD8⁺ cytotoxic T-lymphocytes.

WO 2009/007124 describes the general concept of providing triplebodies. However, this document does not disclose the particular combination of CD123, CD33 and CD16 to achieve the targeted elimination of AML-LSCs for the treatment of AML and myelodysplastic syndrome. To the inventors' best knowledge, neither trispecific antibodies nor recombinant trispecific antibody derivatives connecting CD123 and CD33 on AML cells and CD16 on effector cells have so far been reported and it could not be expected that this particular combination would result in the successful targeting of AML-LSCs in the treatment of AML and myelodysplastic syndrome. Nonetheless, the triplebody of the present invention provides superior elimination of target cells, as shown in example 6 below.

The ratio of at least 2:1 of binding specificities to antigens expressed on AML tumor cells (i.e. CD123 and CD33) and binding specificity to effector cell antigens (i.e. CD16) is an advantageous feature of the present invention. The higher fraction of binding specificities to antigens expressed on leukaemic tumor cells has the effect that the affinity for the tumor cells is increased. Thus, the higher number of antigen binding moieties targeted to antigen expressed on leukaemic tumor cells increases the probability that the molecule of the invention binds to a leukaemic tumor cell before it binds to an effector cell. The recruitment of leukaemic tumor cells prior to the effector cells is advantageous for the following reason: in a therapeutic context, immune responses are generally induced upon binding of an immunoglobulin with one or two specificities or a natural trigger molecule to a respective antigen expressed on the surface of effector cells mediating an immune response. Thereby, the effector cells cannot distinguish whether an antibody molecule is bound to a tumor cell or not, thus leading to an unspecific immune response in case the antibody derivative is not bound to a leukaemic tumor cell. In contrast, the 2:1 ratio of antigen binding sites specific for leukaemic tumor antigens and those specific for effector cells enhance the probability that the molecule of the present invention binds to a leukaemic tumor cell before it binds to an antigen of an effector cell. As a consequence of the enhanced avidity to the leukaemic tumor cells the cell surface retention time on the tumor cells is prolonged, thus resulting in an improved targeting potential. This greatly reduces the amount of unspecific immune responses and may thus decrease adverse side-effects of previously known molecules.

In one embodiment of the molecule of the invention, the binding specificities are conferred by V_{H} and V_{L} domains.

The terms "V_{H} domain" and "V_{L} domain" are used according to the definitions provided in the art. Thus, they refer to the variable region of the heavy chain (V_{H}) and the variable region of the light chain (V_{L}) of immunoglobulins, respectively. Generally, V_{H} and V_{L} domains comprise three complementarity determining regions (CDRs) each, wherein CDRs are highly variable regions mainly responsible for the binding of the antigen. In accordance with the present invention, a V_{L} or V_{H} domain may consist of at least one CDR per domain, preferably at least two or preferably all three CDRs, as long as the resulting immunoglobulin domain exerts the desired function, i.e. specifically binds to its target antigen. Preferably, the target antigens are human antigens, i.e. human CD123, human CD33 and human CD16.
It virill be understood that binding specificities may independently be conferred by only a V_{H} or only a V_{L} domain, or by a combination of both V_{H} and V_{L} domains, for each binding specificity to (a), (b) and (c). Preferably, the binding specificities conferred by V_{H} and V_{L} domains are conferred by a combination, i.e. both V_{H} and V_{L} domains, for each binding specificity to (a), (b) and (c).

In an alternative embodiment of the molecule of the invention, the binding specificities are conferred by anticalins, adnectins, affibodies, or DARPins. Also described herein is that the binding specificities are conferred by ligands.

The term "ligands" refers to molecules that are able to bind to and form a complex with the respective targets, i.e. CD123, CD16 or CD33. The term also refers to fragments and/or derivatives thereof. The naturally occurring ligand for CD123 is interleukin-3 (IL-3), while the naturally occurring ligand for CD16 is the Fc portion of immunoglobulin G and the naturally occurring ligand for CD33 is sialic acid, preferably N-acetylneuraminic acid-α6-galactose-β4-N-acetylglucosamine.
The term "fragments and/or derivatives thereof" refers to fragments of the molecules and/or modified versions of the molecules still having one or more of the biological functions of the full-length molecules. In particular, the fragments and/or derivatives of ligands as described herein are capable of binding the respective antigen CD123, CD16 or CD33. Most preferably the fragments and/or derivatives of ligands are capable of specifically binding the respective antigen CD123, CD16 or CD33.
It is well known in the art that functional molecules, such as for examples (poly)peptides or saccharides may be cleaved to yield fragments with unaltered or substantially unaltered function. Such cleavage may include the removal of a given number of N- and/or C-terminal amino acids from (poly)peptides. Additionally or alternatively, a number of internal (non-terminal) amino acids may be removed, provided the obtained (poly)peptide has the function of the full length (poly)peptide. Said number of amino acids to be removed from the termini and/or internal regions may be one, two, three, four, five, six, seven, eight, nine, ten, 15, 20, 25, 30, 40, 50 or more than 50. Any other number between one and 50 is also deliberately envisaged in accordance with this invention. Similarly, fragments of saccharides include those having deletions at the end as well as those having internal deletions.

Means and methods for determining such functional domains of (poly)peptides or saccharides are well known in the art and include experimental and bioinformatic means. Experimental means include the systematic generation of deletion mutants and their assessment in assays for the desired functions above known in the art. Bioinformatic means include database searches. Suitable databases included protein sequence databases as well as databases for glycobiology, as for example described in von der Lieth (2003). In this case a multiple sequence alignment of significant hits is indicative of domain boundaries, wherein the domain(s) is/are comprised of the/those sub-sequences exhibiting an elevated level of sequence conservation as compared to the remainder of the sequence. Further suitable databases include databases of statistical models of conserved protein domains such as Pfam maintained by the Sanger Institute, UK (www.sanger:ac.uk/Software/Pfam).
It is furthermore known in the art that molecules may be modified in order to obtain derivatives. Such modifications include, without being limiting, (i) esterification of carboxyl groups, or (ii) esterification of hydroxyl groups with carboxylic acids, or (iii) esterification of hydroxyl groups to, e.g. phosphates, pyrophosphates or sulfates or hemi-succinates, or (iv) formation of pharmaceutically acceptable salts, or (v) introduction of hydrophilic moieties, or (vi) introduction/exchange of substituents on aromates or side chains, change of substituent pattern, or (vii) modification by introduction of isosteric or bioisosteric moieties, or (viii) synthesis of homologous compounds, or (ix) introduction of branched side chains, or (x) conversion of alkyl substituents to cyclic analogues, or (xi) derivatisation of hydroxyl group to ketales, acetales, or (xii) N-acetylation to amides, phenylcarbamates, or (xiii) synthesis of Mannich bases, imines, or (xiv) transformation of ketones or aldehydes to Schiff's bases, oximes, acetales, ketales, enolesters, oxazolidines, thiazolidines or combinations thereof.

The various steps recited above are generally known in the art. They include or rely on quantitative structure-action relationship (QSAR) analyses (Kubinyi, "Hausch-Analysis and Related Approachs". VCH Verlag; Weinheim, 1992), combinatorial biochemistry, classical chemistry and others (see, for example, Holzgrabe and Bechtold, Deutsche Apotheker Zeitung 140(8), 813-823, 2000).

The term "anticalins" as used herein refers to engineered proteins derived from lipocalins (Beste *et al*. 1999; Gebauer and Skerra, 2009). Anticalins possess an eight-stranded β-barrel which forms a highly conserved core unit among the lipocalins and naturally forms binding sites for ligands by means of four structurally variable loops at the open end. Anticalins, although not homologous to the IgG superfamily, show features that so far have been considered typical for the binding sites of antibodies: (i) high structural plasticity as a consequence of sequence variation and (ii) elevated conformational flexibility, allowing induced fit to targets with differing shape.

"Adnectins" (also referred to as monobodies) in accordance with the present invention, are based on the 10th extracellular domain of human fibronectin III (10Fn3), which adopts an Ig-like b-sandwich fold of 94 residues with 2 to 3 exposed loops, but lacks the central disulphide bridge (Gebauer and Skerra, 2009).

"Affibodies", in accordance with the present invention, are based on the Z-domain of staphylococcal protein A, a three-helix bundle of about 58 residues providing an interface on two of its α-helices (Gebauer and Skerra, 2009).

In accordance with the present invention, the term "DARPins" refers to designed ankyrin repeat domains (166 residues), which provide a rigid interface arising from typically three repeated b-turns. DARPins usually carry three repeats corresponding to an artificial consensus sequence, whereby six positions per repeat are randomized. Consequently, DARPins lack structural flexibility (Gebauer and Skerra, 2009).

All of the above described binding molecules are well known to the skilled person and are defined in accordance with the prior art and the common general knowledge of the skilled person.

In a preferred embodiment of the molecule of the invention, the binding portions conferring the specificities to (a), (b) and (c) are polypeptides.

In a more preferred embodiment, the molecule of the invention is a single polypeptide chain.

In another preferred embodiment of the molecule of the invention, the binding portions of the molecule conferring the specificities to (a), (b) and (c) are linked by a linker.

The term "linker" as used in accordance with the present invention relates to a sequel of amino acids (i.e. peptide linkers) as well as to non-peptide linkers, which separate the binding portions of the molecule of the invention conferring the specificities to (a) CD123, (b) CD16 and (c) CD33.

Peptide linkers as envisaged by the present invention are (poly)peptide linkers of at least 1 amino acid in length. Preferably, the linkers are 1 to 100 amino acids in length. More preferably, the linkers are 5 to 50 amino acids in length and even more preferably, the linkers are 10 to 20 amino acids in length. The linkers separating the three binding portions of the molecule of the invention can have the same or different lengths and may comprise the same or a different amino acid sequence. In one preferred embodiment, the linkers have the same length and the same amino acid sequence.

In another preferred embodiment, the linkers separating the three binding portions of the molecule of the invention differ in length and/or amino acid sequence from each other. Furthermore, the nature, i.e. the length and/or amino acid sequence of the linker may modify or enhance the stability and/or solubility of the molecule. The length and sequence of a linker depends on the composition of the respective binding portions of the molecule of the invention.
The skilled person is well aware of methods to test the suitability of different linkers. For example, the properties of the molecule can easily be tested by comparing the binding affinity of the binding portions of the molecule of the invention. In case of the tri-specific molecule of the invention, the respective measurements for each binding portion may be carried out. The stability of the resulting molecule can be measured using a flow cytometry based method to determine the residual binding capacity of the molecule after incubation in human serum at 37°C for several time periods. Other suitable tests can e.g. be found in Bruenke et al. (2005).

In a preferred embodiment, the linker is a flexible linker using e.g. the amino acids alanine and serine or glycine and serine. Preferably the linker sequences are (Gly₄Ser)₄, or (Gly₄Ser)₃.

It will be appreciated by the skilled person that when the molecule of the invention is a single polypeptide chain, the linker is a peptide linker.

The term "non-peptide linker", as used in accordance with the present invention, refers to linkage groups having two or more reactive groups but excluding peptide linkers as defined above. For example, the non-peptide linker may be a polymer having reactive groups at both ends, which individually bind to reactive groups of the binding portions of the molecule of the invention, for example, an amino terminus, a lysine residue, a histidine residue or a cysteine residue. The reactive groups of the polymer include an aldehyde group, a propionic aldehyde group, a butyl aldehyde group, a maleimide group, a ketone group, a vinyl sulfone group, a thiol group, a hydrazide group, a carbonyldimidazole (CDI) group, a nitrophenyl carbonate (NPC) group, a trysylate group, an isocyanate group, and succinimide derivatives. Examples of succinimide derivatives include succinimidyl propionate (SPA), succinimidyl butanoic acid (SBA), succinimidyl carboxymethylate (SCM), succinimidyl succinamide (SSA), succinimidyl succinate (SS), succinimidyl carbonate, and N-hydroxy succinimide (NHS). The reactive groups at both ends of the non-peptide polymer may be the same or different. For example, the non-peptide polymer may have a maleimide group at one end and an aldehyde group at another end.

In a further preferred embodiment, the linker is a peptide linker.

The present invention relates in a further preferred embodiment to a molecule of the invention, wherein the molecule comprises a first immunoglobulin domain comprising a V_{L} domain linked to a V_{H} domain, wherein the immunoglobulin domain specifically binds to CD123; a second immunoglobulin domain comprising a V_{L} domain linked to a V_{H} domain, wherein the immunoglobulin domain specifically binds to CD16; and a third immunoglobulin domain comprising a V_{L} domain linked to a V_{H} domain, wherein the immunoglobulin domain specifically binds to CD33.
It will be understood by one of skill in the art that said first immunoglobulin domain that specifically binds to CD123 relates to the binding specificity recited in (a) of the molecule of the invention above. Similarly, said second immunoglobulin domain that specifically binds to CD16 relates to the binding specificity recited in (b) of the molecule of the invention above and said third immunoglobulin domain that specifically binds to CD33 relates to the binding specificity recited in (c) of the molecule of the invention above.

The term "immunoglobulin domain comprising a V_{L} domain linked to a V_{H} domain" as used in accordance with the present invention refers to single-chain fragment variable domains of immunoglobulins (scFv), which have been shown to be necessary and sufficient to bind their antigen. The V_{L} domains are linked to the V_{H} domains, i.e. they are connected with each other either directly or via a linker.

In accordance with the present invention, the molecule of the invention may comprise immunoglobulin, domains derived from a single species, but may also be a chimeric or humanized molecule.

The immunoglobulin domains of (a) to (c) of the molecule of the invention may be arranged in any order, such as for example (a)-(b)-(c), (b)-(c)-(a), (c)-(a)-(b), (b)-(a)-(c) etc. More preferably, the immunoglobulin domains of (a) to (c) are arranged in the recited order (i.e. (a)-(b)-(c)) either in the N-terminus to C-terminus direction or, alternatively, in the C-terminus to N-terminus direction. Preferably, the immunoglobulin domains of (a) to (c) are arranged in the recited order in the N-terminus to C-terminus direction. The V_{L} and V_{H} domain may be arranged within the immunoglobulin domain such that the V_{L} domain is positioned N- or C-terminal of the V_{H} domain. Accordingly, in a nucleic acid molecule encoding the molecule of the present invention, the nucleic acid sequence encoding the V_{L} domain may be positioned 5' or 3' of the nucleic acid sequence encoding the V_{H} domain. The skilled person is able to determine which arrangement of the V_{H} and V_{L} domains is more suitable for a specific scFv. Preferably, the V_{L} domain is positioned N-terminal of the V_{H} domain.

In a preferred embodiment of the invention, at least one immunoglobulin domain comprises at least two cysteine residues capable of forming intramolecular disulfide bridges.

In accordance with the present invention, immunoglobulin domains binding to antigens of interest may be stabilized by the formation of at least one intramolecular disulfide bridge. Suitable cysteine residues may be naturally occurring in the immunoglobulin domains or may be introduced into the immunoglobulin domains by mutating appropriate amino acids to cysteine. The following prerequisites have to be met when designing a modified antibody with artificial cysteine bridges. First, the disulfide bridge should connect amino acids in structurally conserved regions of the Fv, i.e. in the V_{H} and V_{L} regions of immunoglobulins. In other words, the disulfide bridge forms between a cysteine in V_{H} and a cysteine in V_{L} within one of the immunoglobulin domains (a) to (c) according to the method of the invention. A disulfide bridge may be formed in more than one of these immunoglobulin domains, however, not between different immunoglobulin domains. Furthermore, the distance between the V_{H} and the V_{L} domain should be small enough to enable the formation of a disulfide bridge without the generation of adverse strain in the Fv molecule. Finally, the disulfide bridge should be distant enough from the CDRs to not interfere with antigen binding. As a consequence, cysteines introduced to form the disulfide bridge should be in the framework regions of the Fv which connect the CDRs of the V_{H} and V_{L} regions at positions that are structurally conserved in most antibodies. Glockshuber et al. (1990), Brinkmann et al. (1993) and Reiter et al. (1994) set out these criteria and successfully established the first disulfide stabilized Fv antibodies. Mutations in the amino acid sequence of the molecule of the invention can e.g. be introduced according to Reiter et al. (1994) in a nucleic acid molecule encoding said molecule at base triplets at position 100 in the V_{L} domain or at position 44 in the V_{H} domain, so that they encode a cysteine residue. All positions are according to the Kabat numbering (Kabat et al., 1991). Means to introduce mutations into a nucleic acid molecule are known to the skilled person and can e.g. be retrieved from Sambrook and Russell "Molecular Cloning, A Laboratory Manual", Cold Spring Harbor Laboratory, N.Y. (2001) and Ausubel, Current Protocols in Molecular Biology, Green Publishing Associates and Wiley Interscience, N.Y. (2001). Preferably, a disulfide bridge forms between a cysteine in the V_{H} and a cysteine in the V_{L} region of the immunoglobulin domain of (b) according to the molecule of the invention.

As has been disclosed in WO 2009/007124, it was previously shown that the introduction of disulfide bridges into immunoglobulin domains of scFvs greatly enhances the stability of these molecules, without affecting their specificity or reducing their affinity and functionality and without a loss of solubility of the molecule.

In a further preferred embodiment, the molecule of the invention comprises at least one linker which separates at least one V_{H} from a V_{L} domain or at least one V_{L} from a V_{H} domain.

The linkers separating the V_{H} and V_{L} domains of one immunoglobulin domain and the linkers separating different immunoglobulin domains can have the same or different lengths and may be selected to comprise the same or a different composition, for example they may comprise the same or a different amino acid sequence or non-peptide polymer. In one preferred embodiment, the linkers have the same length and the same composition.

In another preferred embodiment, the linkers separating different immunoglobulin domains differ in length and/or composition from the linkers separating the V_{H} and V_{L} regions within an immunoglobulin domain. For example, the former could be longer and designed in order to promote the flexibility of the immunoglobulin domains towards each other or to facilitate correct folding of the molecule and/or enhance the affinity of one immunoglobulin domain to its target antigen. Furthermore, the nature, i.e. the length and/or composition of the linker may modify or enhance the stability and/or solubility of the molecule.

As mentioned above, the length and sequence of a linker depends on the composition of the respective V_{H} and V_{L} domains forming an immunoglobulin domain. For example, starting from the N-terminus of the polypeptide of the invention, if the V_{L} domain is followed by the V_{H} domain, linkers of 20 amino acid separating the V regions of one immunoglobulin domain may be optimal. In contrast, if the V_{H} domain is followed by the V_{L} domain, the respective linker my have an optimal length of 15 amino acids. Without wishing to be bound by any scientific theory, it is believed that these differences may be due to sterical reasons leading to linkers of different lengths promoting the correct folding of immunoglobulin domains having a different arrangement of V domains.
The skilled person is well aware of methods to test the suitability of different linkers within or between immunoglobulin domains, for example by employing any of the methods described above.

In a preferred embodiment, at least two variable domains are fused by a flexible linker using e.g. the amino acids alanine and serine or glycine and serine. Preferably the linker sequences are (Gly₄Ser)₄, or (Gly₄Ser)₃.

In another preferred embodiment no linker is present between at least one V_{H} and V_{L} domain or V_{L} and V_{H} domain within or between immunoglobulin domains.

In another preferred embodiment, all variable domains are fused by a linker.

In another preferred embodiment, the molecule of the invention further comprises at least one additional (poly)peptide.

Preferably, the additional (poly)peptide is unrelated to immunoglobulin domains and can be, for example, a tag or a functional (poly)peptide suitable to improve the performance of the polypeptide of the invention. The tag can e.g. be a Strep-tag, a His-tag, a Myc-tag or a Flag-tag. Functional (poly)peptides are e.g. a kappa secretion leader, human serum albumin, (hsa) or fragments thereof, (poly)peptides capable of binding to hsa or other serum proteins; (poly)peptides capable of binding to neonatal Fc receptor (FcRn), human muscle aldolase (hma) or fragments thereof, CD8 hinge region, immunoglobulin constant regions, Interleukin-2, Interleukin-15 and Interleukin-18, Granulocyte-Macrophage-Colony Stimulating Factor (GM-CSF), Granulocyte Stimulating Factor (G-CSF) or a (poly)peptide providing at least one N-glycosylation site.

The term "fragments thereof" is as defined above. In particular, the fragments of (poly)peptides as envisaged in this embodiment are capable of increasing the stability and/or the serum half-life of the antibody derivative of the present invention.

Some of the (poly)peptides to be further encoded by the nucleic acid molecule of the present invention may facilitate the purification of the recombinantly expressed polypeptide, e.g. various tags. Methods to add tags and/or other (poly)peptides to the polypeptide encoded by the nucleic acid molecule of the present invention are well known to the skilled person and described e.g. in Sambrook, 2001, loc. cit.

In a preferred embodiment of the invention, the first immunoglobulin domain comprises an amino acid sequence selected from the group consisting of SEQ ID NOs: 2, 4, 6, 8, 10, 12, 14, 16, 18, 20, 22, 24 and 26.

In a further preferred embodiment of the invention, the first immunoglobulin domain comprises the amino acid sequence of SEQ ID NO: 2, representing a preferred form of the CD123-specific scFv.

In another preferred embodiment of the invention, the molecule of the invention has the amino acid sequence shown in SEQ ID NO: 28.

The present invention further relates to a nucleic acid molecule encoding the molecule of the invention.

The nucleic acid molecule may encode the entire molecule, i.e. where the molecule is a single-chain polypeptide or, alternatively, may encode the individual binding portions of the molecule of the invention, where these binding portions are of a proteinaceous nature. Upon expression of these binding portions, they may form the molecule of the invention via noncovalent bonds such as for example hydrogen bonds, ionic bonds, van der Waals forces or hydrophobic interacts or via covalent bonds such as for example disulfide bonds.

Preferably, the polypeptide encoded by the nucleic acid molecule of the invention is a single-chain polypeptide.

In accordance with the present invention the term "nucleic acid molecule" defines a linear molecular chain consisting of more than 30 nucleotides. The group of molecules designated herein as "nucleic acid molecules" also comprises complete genes.

"Nucleic acid molecules", in accordance with the present invention, include DNA, such as for example cDNA or genomic DNA, and RNA, for example mRNA. Further included are nucleic acid mimicking molecules known in the art such as for example synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. They may contain additional non-natural or derivative nucleotide bases, as will be readily appreciated by those skilled.in the art.
The present invention furthermore contemplates nucleic acid molecules complementary to the above-defined nucleic acid molecule as well as nucleic acid molecules hybridizing thereto under stringent conditions.

It is well known in the art how to perform hybridization experiments with nucleic acid molecules. Correspondingly, the person skilled in the art knows what hybridization conditions he/she has to use to allow for successful hybridization. The establishment of suitable hybridization conditions is referred to in standard text books such as Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985).
"Stringent conditions" refer to hybridization conditions which allow nucleic acid molecules capable of hybridizing to the nucleic acid molecules of the invention or parts thereof to hybridize to these target sequences to a detectably greater degree than to other sequences (e.g. at least 2-fold over background). Stringent conditions are sequence-dependent and will differ depending on the circumstances. By controlling the stringency of the hybridization and/or washing conditions, target sequences that have at least 90% sequence identity, more preferably 95%, such as 98% and more preferably 100% sequence identity to the respective probe, i.e. the nucleic acid molecule of the invention, can be identified (highly stringent hybridization conditions). Alternatively, stringency conditions can be adjusted to allow a higher degree of mismatching in sequences (low stringency conditions of hybridization). Such highly stringent and low stringent conditions for hybridization are well known to the person skilled in the art. The embodiment recited herein above preferably refers to highly stringent conditions. For example, highly stringent conditions for hybridization comprise e.g. an overnight incubation at 65°C in 4x SSC (600 mM NaCl, 60 mM sodium citrate) followed by washing at 65°C in 0.1x SSC for one hour. Alternatively, highly stringent hybridization conditions can comprise an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing in e.g. 0.1-0.5x SSC at about 55-65°C for about 5 to 20 min.
Changes in the stringency of hybridization are primarily accomplished through the manipulation of formamide concentration (lower percentages of formamide result in lowered stringency), salt conditions, or temperature. For example, lower stringency conditions include an overnight incubation at 50°C in 4x SSC or an overnight incubation at 37°C in a solution comprising 6x SSPE (20x SSPE = 3M NaCl; 0.2M NaH₂PO₄; 0.02M EDTA, pH 7.4), 0.5% SDS, 30% formamide, 100 mg/ml salmon sperm blocking DNA; followed by washes at 50°C with 1x SSPE, 0.1% SDS. In addition, to achieve an even lower stringency, washes performed following stringent hybridization can be done at higher salt concentrations (e.g. 5x SSC). It is of note that variations in the above conditions may be accomplished through the inclusion and/or substitution of alternate blocking reagents. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed).

In a preferred embodiment of the nucleic acid molecule of the invention, the nucleic acid molecule comprises (i) the nucleic acid sequence as shown in SEQ ID NO: 27; (ii) a nucleic acid sequence encoding the amino acid sequence shown in SEQ ID NO: 28; or (iii) a nucleic acid sequence that is degenerate with respect to the nucleic acid molecule of (i) or (ii).

The present invention also relates to a vector comprising the nucleic acid molecule of the invention.

Preferably, the vector is a plasmid, cosmid, virus, bacteriophage or another vector used e.g. conventionally in genetic engineering.
The nucleic acid molecule of the present invention may be inserted into several commercially available vectors. Non-limiting examples include prokaryotic plasmid vectors, such as the pUC-series, pBluescript (Stratagene), the pET-series of expression vectors (Novagen) or pCRTOPO (Invitrogen) and vectors compatible with expression in mammalian cells like pREP (Invitrogen), pSecTag2HygroC (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Intvitrogen).
The nucleic acid molecule of the present invention referred to above may also be inserted into vectors such that a translational fusion with another nucleic acid molecule is generated. The other nucleic acid molecules may encode a (poly)peptide which can e.g. increase the solubility and/or facilitate the purification of the protein encoded by the nucleic acid molecule of the invention. Non-limiting examples include pET32, pET41, pET43 (Novagen). The vectors may also contain an additional expressible polynucleotide coding for one or more chaperons to facilitate correct protein folding. Suitable bacterial expression hosts comprise e. g. strains derived from BL21 (such as BL21(DE3), BL21(DE3)PlysS, BL21(DE3)RIL, BL21 (DE3)PRARE) or Rosetta®.
For vector modification techniques, see Sambrook and Russel, 2001, loc. cit. Generally, vectors can contain one or more origin of replication (ori) and inheritance systems for cloning or expression, one or more markers for selection in the host, e. g., antibiotic resistance, and one or more expression cassettes. Suitable origins of replication (ori) include, for example, the Col E1, the SV40 viral and the M 13 origins of replication.
A typical mammalian expression vector contains the promoter element, which mediates the initiation of transcription of mRNA, the protein coding sequence, and signals required for the termination of transcription and polyadenylation of the transcript. Moreover, elements such as origin of replication, drug resistance gene, regulators (as part of an inducible promoter) may also be included. The *lac* promoter is a typical inducible promoter, useful for prokaryotic cells, which can be induced using the lactose analogue *isopropylthiol-b-D-galactoside*. ("IPTG"). For recombinant expression, the antibody fragment may be ligated between e.g. the PeIB leader signal, which directs the recombinant protein in the periplasm and the gene III in a phagemid called pHEN4 (described in Ghahroudi *et al*, 1997). Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). The co-transfection with a selectable marker such as dhfr, gpt, neomycin, hygromycin genes for eukaryotic cells or tetracycline, kanamycin or ampicillin resistance genes for culturing in *E. coli* and other bacteria allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al. 1991; Bebbington et al. 1992). Using these markers, the mammalian cells are grown in selective medium and the cells with the highest resistance are selected.

Possible regulatory elements permitting expression in prokaryotic host cells comprise, e.g., the *lac*, *trp* or *tac* promoter, the lacUV5 or the trp promotor in *E*. *coli,* and examples for regulatory elements permitting expression in eukaryotic host cells (the more preferred embodiment) are the *AOX1* or *GAL1* promoter in yeast or the CMV- (Cytomegalovirus), SV40-, RSV-promoter (Rous sarcoma virus), the gai10 promoter, human elongation factor 1α-promoter, CMV enhancer, CaM-kinase promoter, the Autographa californica multiple nuclear polyhedrosis virus (AcMNPV) polyhedral promoter or a globin intron in mammalian and other animal cells. Preferred promoters are natural immunoglobulin promoters. Besides elements which are responsible for the initiation of transcription such regulatory elements may also comprise transcription termination signals, such as the SV40-poly-A site or the tk-poly-A site or the SV40, lacZ and AcMNPV polyhedral polyadenylation signals, downstream of the polynucleotide.
The coding sequences inserted in the vector can e.g. be synthesized by standard methods, or isolated from natural sources or produced semi-synthetically, i.e. by combining chemical synthesis and recombinant techniques. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e. g., translation initiation codon, promoters, enhancers, and/or insulators), internal ribosomal entry sites (IRES) (Owens *et al*., 2001) and optionally poly-A signals ensuring termination of transcription and stabilization of the transcript. Additional regulatory elements may include transcriptional as well as translational enhancers, and/or naturally-associated or heterologous promoter regions. Preferably, the nucleic acid molecule of the invention is operably linked to such expression control sequences allowing expression in prokaryotes or eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.
Specifically-designed vectors allow the shuttling of DNA between different hosts, such as bacteria- fungal cells or bacteria-animal cells.
An expression vector according to this invention is capable of directing the replication, and the expression, of the polynucleotide and encoded enzyme of this invention. Suitable expression vectors which comprise the described regulatory elements are known in the art such as Okayama-Berg cDNA expression vector pcDV1 (Pharmacia), pRc/CMV, pcDNA1, pcDNA3, pSecTag2HygroC (Invitrogen, as used, inter alia in the appended examples), pSPORT1 (GIBCO BRL) or pGEMHE (Promega), or prokaryotic expression vectors, such as lambda gt11, pJOE, the pBBR1-MCS -series, pJB861, pBSMuL, pBC2, pUCPKS, pTACT1 or, preferably, the pET vector (Novagen).

The nucleic acid molecules of the invention as described herein above may be designed for direct introduction or for introduction via liposomes, phage vectors or viral vectors (e.g. adenoviral, retroviral) into the cell. Additionally, baculoviral systems or systems based on Vaccinia Virus or Semliki Forest Virus can be used as eukaryotic expression system for the nucleic acid molecules of the invention. Expression vectors derived from viruses such as retroviruses, vaccinia virus, adeno-associated virus, herpes viruses, or bovine papilloma virus, may be used for delivery of the polynucleotides or vector into targeted cell population. Methods which are well known to those skilled in the art can be used to construct recombinant viral vectors; see, for example, the techniques described in Sambrook, 2001 and Ausubel, 2001, loc. cit.

In another preferred embodiment the present invention relates to a non-human host transformed with the vector of the invention.

Said host may be produced by introducing the vector of the invention into a host, which upon its presence mediates the expression of the polypeptide encoded by the vector.
In accordance with the present invention, the host may be a transgenic non-human animal transfected with and/or expressing the vector of the present invention. In a preferred embodiment, the transgenic animal is a mammal, e.g. a hamster, cow, cat, pig, dog or horse.

In a preferred embodiment, the host is a cell, such as an isolated cell which may be part of a cell culture.

Suitable prokaryotic host cells comprise e.g. bacteria of the species Escherichia, *Bacillus, Streptomyces* and *Salmonella typhimuriurri.* Suitable eukaryotic host cells are e.g. fungal cells, inter alia, yeasts such as *Saccharomyces cerevisiae* or *Pichia pastoris* or insect cells such as Drosophila S2 and Spodoptera Sf9 cells and plant cells as well as mammalian cells. Appropriate culture mediums and conditions for the above-described host cells are known in the art.
Mammalian host cells include without being limiting human Hela, HEK293, H9 and Jurkat cells, mouse NIH3T3 and C127 cells, COS 1, COS 7 and CV1, quail QC1-3 cells, mouse L cells, Chinese hamster ovary (CHO) cells and Bowes melanoma cells. Alternatively, the recombinant polypeptide of the invention can be expressed in stable cell lines that contain the gene construct encompassing the nucleic acid molecule or the vector of the invention integrated into a chromosome.
In a more preferred embodiment, said cell is a primary cell or primary cell line. Primary cells are cells which are directly obtained from an organism. Suitable primary cells are, for example, mouse embryonic fibroblasts, mouse primary hepatocytes, cardiomyocytes and neuronal cells as well as mouse muscle stem cells (satellite cells) and stable, immortalized cell lines derived thereof.

The present invention also relates to a method for the production of a polypeptide comprising culture of the host cell of the invention under suitable conditions and isolation of the recombinant polypeptide produced.

Suitable conditions for culturing a prokaryotic or eukaryotic host are well known to the person skilled in the art. For example, suitable conditions for culturing bacteria are growing them under aeration in Luria Bertani (LB) medium. To increase the yield and the solubility of the expression product, the medium can be buffered or supplemented with suitable additives known to enhance or facilitate both. *E. coli* can be cultured from 4 to about 37 °C, the exact temperature or sequence of temperatures depends on the molecule to be over-expressed. In general, the skilled person is also aware that these conditions may have to be adapted to the needs of the host and the requirements of the polypeptide expressed. In case an inducible promoter controls the nucleic acid molecule of the invention in the vector present in the host cell, expression of the polypeptide can be induced by addition of an appropriate inducing agent. Suitable expression protocols and strategies are known to the skilled person.

Depending on the cell type and its specific requirements, mammalian cell cultures can e.g. be carried out in RPMI or DMEM medium containing 10% (v/v) FCS, 2mM L-glutamine and 100 U/ml penicillin/streptomycine. The cells can be kept at 37 °C in a 5% CO₂, water saturated atmosphere.
Suitable media for insect cell culture is e.g. TNM + 10% FCS or SF900 medium. Insect cells are usually grown at 27 °C as adhesion or suspension culture.
Suitable expression protocols for eukaryotic cells are well known to the skilled person and can be retrieved e.g. from in Sambrook, 2001, loc cit.

Methods of isolating the polypeptide produced are well-known in the art and comprise without being limiting method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, in Sambrook, 2001, loc. cit.

The present invention also relates to a diagnostic composition comprising at least one of the molecules of the invention, preferably a polypeptide, the nucleic acid molecules of the invention or the vector of the invention.

The enhanced affinity and/or avidity of the molecule of the invention enables its use in diagnostic assays. For example, the molecule can be used as a sensitive detection agent for the detection of AML-LSCs, as these express CD123 and CD33 on the cell surface. Especially the high stability of the disulfide stabilized CD16-specific scFv (also referred to herein as ds16) and the naturally highly stable CD123- and CD33-specific scFvs, which are incorporated in this format, allow a long time storage, which is desirable for diagnostic agents.

Furthermore, the present invention relates to a pharmaceutical composition comprising at least one of the molecules of the invention, preferably a polypeptide, the nucleic acid molecule of the invention or the vector of the invention.

In accordance with the present invention, the term "pharmaceutical composition" relates to a composition for administration to a patient, preferably a human patient. The pharmaceutical composition of the invention comprises the compounds recited above (wherein the term "compound" refers to the mentioned molecule, nucleic acid molecule, and the vector as well as fragments or derivatives or modifications thereof), alone or in combination. It may, optionally, comprise further molecules capable of altering the characteristics of the compounds of the invention thereby, for example, stabilizing, modulating and/or activating their function. The composition may be in solid, liquid or gaseous form and may be, *inter alia,* in the form of (a) powder(s), (a) tablet(s), (a) solution(s) or (an) aerosol(s). The pharmaceutical composition of the present invention may, optionally and additionally, comprise a pharmaceutically acceptable carrier. By "pharmaceutically acceptable carrier" is meant a non-toxic solid, semisolid or liquid filler, diluent, encapsulating material or formulation auxiliary of any type. Examples of suitable pharmaceutically acceptable carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known conventional methods. The term "parenteral" as used herein refers to modes of administration, which include intravenous, intramuscular, intraperitoneal, intrasternal, subcutaneous and intraarticular injection and infusion. These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day.

As mentioned above, the nucleic acid molecule of the invention may be formulated into a pharmaceutical composition. The nucleic acid molecule is eventually to be introduced into the desired cells. Appropriate formulations include those wherein 10⁶ to 10¹² copies of the DNA molecule, advantageously comprised in an appropriate vector are administered per dose. The vector may be, for example, a phage, plasmid, viral or retroviral vector. Retroviral vectors may be replication competent or replication defective. In the latter case, viral propagation generally will occur only in complementing host/cells.

The invention also relates to the molecule of the invention, preferably a polypeptide, the nucleic acid molecule of the invention or the vector of the invention for use in the treatment of acute myeloid leukaemia and/or myelodysplastic syndrome.

The term "acute myeloid leukaemia" (AML) is used in accordance with the definitions provided in the art. Thus, it refers to a cancer of the myeloid line of blood cells, characterized by the rapid growth of abnormal white blood cells that accumulate in the bone marrow and interfere with the production of normal blood cells. In later stages, abnormal white blood cells also accumulate in the blood stream.

The term "myelodysplastic syndrome" (MDS) is used in accordance with the definitions provided in the art. Thus, it refers to a bone marrow stem cell disorder resulting in disorderly and ineffective hematopoiesis manifested by irreversible quantitative and qualitative defects in hematopoietic cells. In a majority of cases, the course of disease is chronic with gradually worsening cytopenias due to progressive bone marrow failure. Approximately one-third of patients with MDS progress to AML within months to a few years.

The molecule of the invention, the nucleic acid molecule of the invention or the vector of the invention can be used for the treatment of acute myeloid leukaemia and/or myelodysplastic syndrome. Therefore, the present invention also relates to a method of treating acute myeloid leukaemia and/or myelodysplastic syndrome comprising the administration of an amount of the molecule of the invention, preferably a polypeptide, the nucleic acid of the invention or the vector of the invention that is effective to exert the desired effect to a patient in need thereof. The desired effect in connection with methods of treatment is inducing an immune response against cells expressing the CD123 and CD33 antigens, especially AML leukaemia stem cells (AML-LSCs).

The invention further relates to the molecule of the invention, preferably a polypeptide, the nucleic acid molecule of the invention or the vector of the invention for use in the treatment of acute myeloid leukaemia and/or myelodysplastic syndrome, wherein the nucleic acid molecule, vector or polypeptide is to be administered in a remission phase for acute myeloid leukaemia or after diagnosis of myelodysplastic syndrome.

The term "remission" is used in accordance with the definitions provided in the art. Thus, it refers to the state of absence of disease activity in patients with a chronic illness, when it may be expected that the illness will manifest again in the future. In accordance with the present invention, it is used to refer to the absence of acute myeloid leukaemia after treatment thereof.

As outlined above, in remission after an initial course of conventional chemotherapy, the blast counts are reduced in the patients bone marrow to approximately 5% or less of total bone marrow leukocytes, and the cells that survived the chemotherapy are referred to as "minimal residual disease" (MRD) cells. These cells are enriched in AML leukaemia stem cells (AML-LSCs), which are particularly resistant to chemotherapy, and constitute a particularly dangerous reservoir of cells capable of re-expanding and causing a relapse, as described above. In the remission stage, normal healthy leukocytes are initially few in numbers, because most have been eliminated by the chemotherapy. However, after a few weeks normal myelopoiesis resumes and the first normal leukocytes to be reconstituted from the surviving healthy hematopoietic stem cells (HSCs) are the granulocytes (PMNs, polymorphonuclear granulocytes) followed by natural killer (NK-) cells, monocytes/macrophages, B-lymphocytes and the last to re-appear are the T-lymphocytes. Granulocytes become quickly available in sufficient numbers, as is evident because patients do not typically show a significantly increased risk for bacterial infections. As granulopoiesis and the reconstitution of other myeloid effector cells occur in a dose-temporal correlation, NK-cells, monocytes and macrophages become available. Therefore, CD16-positive effector cells are available at this stage in sufficient numbers to be recruited by the molecule of the present invention (sctb [123 x disulfide stabilized ds16 x 33] for the elimination of AML-MRD (i.e. AML-LSC) cells.

In a mouse model of human Non-Hodgkin lymphoma (NHL), treated with CD20 and CD19 antibodies, monocytes were reported to be the most relevant population of effector cells (Tedder *et al,* 2006). In addition, the *in vitro* studies provided in the Examples below provide clear evidence that the polypeptide of the present invention activates NK-cells to eliminate AML cells. Nonetheless, it is expected that in a human patient all of the above recited populations of effector cells will contribute to the overall therapeutic effect. This overall effect is not only the result of direct elimination of the tumor cell by antibody dependant cellular cytotoxicity (ADCC, also referred to as "redirected lysis"), but the apoptotic fragments of AML cells resulting from their lysis are taken up by phagocytes and are processed and presented again by CD4⁺ and CD8⁺ T-lymphocytes. Both mechanisms help to build up a secondary titer of humoral anti-tumor antibodies and a cellular anti-tumor response by CD8⁺ cytotoxic T-lymphocytes.

The molecule, the nucleic acid molecule or the vector of the invention are thus considered to contribute a significant therapeutic effect, well beyond the effects possible by previous compounds such as Mylotarg and conventional chemotherapy, while at the same time being accompanied by far fewer side effects, as no chemical, bacterial or plant toxins are involve.

The figures show:

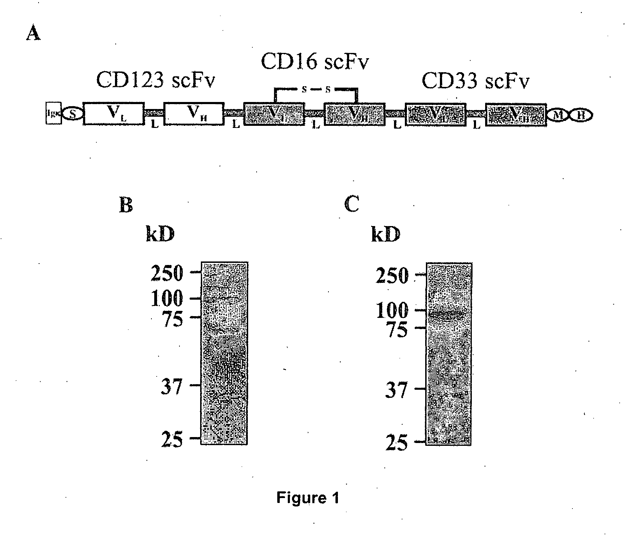

Figure 1. Construction and expression of the single chain triplebodie (sctb) [123 x ds16 x 33]. (A) Design of the sctb [123 x ds16 x 33]. The two distal scFvs are specific for the tumor antigens CD123 and CD33, the central scFv is directed against the activating trigger molecule CD16. Igκ, secretion leader sequence from the murine Ig kappa L chain; V_{L},V_{H}, cDNA sequences coding for the V regions of Ig L-or H-chains; L, cDNA coding for a 20 amino acid flexible linker, e.g. (Gly₄Ser)₄; S, H, M, STREP-, hexahistidine- and myc-tag; S-S, stabilizing disulphide bond. Integrity and purity of the sctb [123 x ds16 x 33] after affinity chromatography with Ni-NTA agarose beads as evaluated by reducing SDS-PAGE and staining with Coomassie blue (B) and Western Blot analysis using an anti-His antibody for detection (C).
Figure 2. Specific antigen binding of the sctb [123 x ds16 x 33].
   FACS analysis of specific binding of the sctb to U937 cells (I), CD123-transfected 293 cells (II), untransfected 293 cells (III), CD16 transfected CHO cells (IV), and untransfected CHO cells (V). White: control sctb; black: sctb [123 x ds16 x 33].

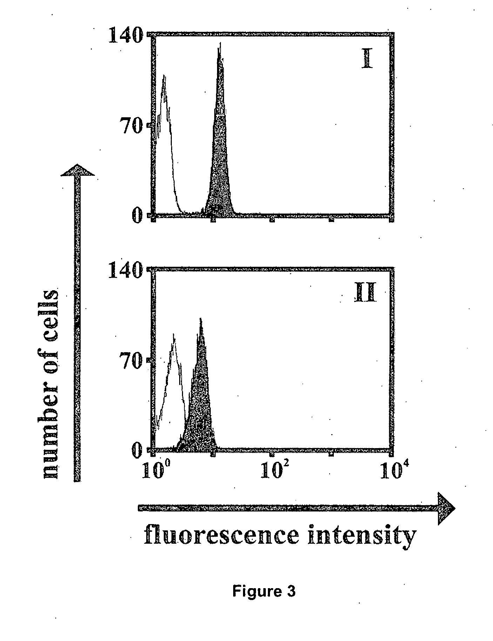

Figure 3. Simultaneous antigen binding of the sctb [123 x ds16 x 33].
   U937 cells were incubated with the sctb and simultaneous binding was revealed by addition of the soluble proteins consisting of the extracellular domain of CD16 and GFP (CD16ex-GFP) (I, measured in the FL1 channel) and the extracellular domain of CD123 and RFP (CD123ex-RFP) (II, measured in the FL2 channel).

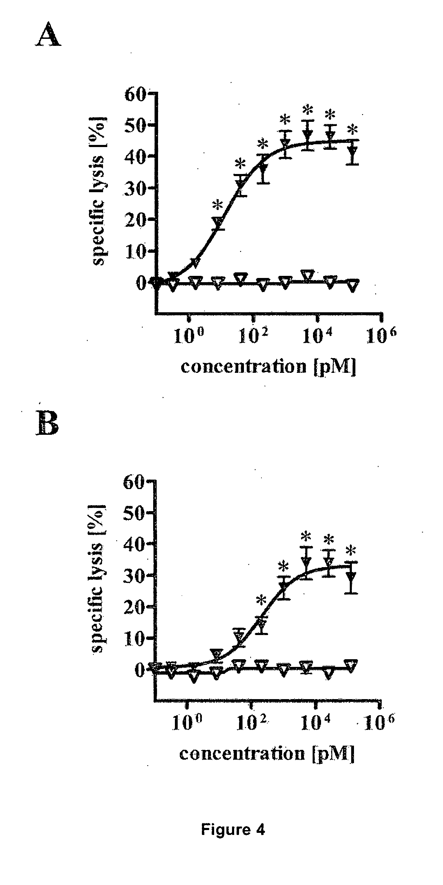

Figure 4. Dose dependent induction of ADCC of different tumor cell lines by the sctb [123 x ds16 x 33].
   The CD123/CD33 double-positive tumor cell lines MOLM-13 (A) and THP-1 (B) were used as targets to compare the efficacy of the sctb at a constant E:T cell ratio of 40:1. The sctb [123 x ds16 x 33] (filled triangle) triggered ADCC in a dose-dependent manner. The non-relevant control sctb (open black triangle) did not induce significant killing. Data points represent mean percentage of lysis + SEM obtained with isolated MNCs from at least four different healthy donors. *Statistically significant differences in ADCC compared to the control sctb.

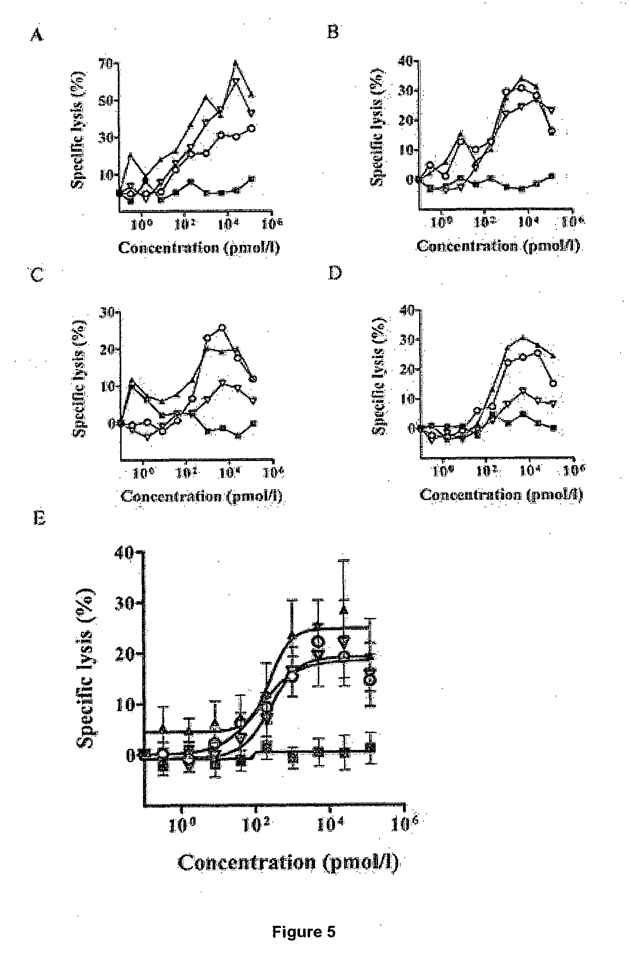

Figure 5. Lysis of primary AML cells by sctbs [123 x ds16 x 33], [123 x ds16 x 123] and [33 x ds16 x 33]. Sctbs [123 x ds16 x 33] (closed triangle), [123 x ds16 x 123] (open circle) and [33 x ds16 x 33] (open triangle) mediated dose-dependent ADCC of primary AML cells, whereas a control sctb (closed square) failed to induce cellular lysis. (A and B) Induction of ADCC by sctbs of purified primary AML cells isolated from peripheral blood (patient 1 and 2). (C and D) Induction of ADCC by sctbs of purified primary AML cells isolated from bone marrow (patient 6 and 7). Data points represent percentage of specific lysis obtained with isolated MNCs from one healthy donor at an E:T ratio of 40:1. (E) Induction of ADCC by sctbs of purified AML cells from peripheral blood of 6 different patients (patient 1-6), combined data. Data points represent mean percentage of specific lysis averaged over the 6 patients obtained with isolated MNCs from one healthy donor per patient sample at an E:T ratio of 40:1. Specific lysis is total lysis minus spontaneous lysis.

The examples illustrate the invention:

### Example 1: Cell lines and hybridomas

Chinese hamster ovary (CHO) cells, stably transfected with a human CD16 cDNA expression vector, were provided by Dr. J. van de Winkel (University Medical Centre, Utrecht, the Netherlands). The hybridoma 3G8; FcγRIII, CD16; IgG1, (Fleit *et al,* 1982) and the human 293T cells were from the American Type Cell Culture Collection (ATCC, Manassas, VA, USA). The human AML cell lines MOLM-13, THP-1 (t(9;11)(p22;q23), expressing *MLL-AF9*) and U937 were from the German Collection of Microorganisms and Cell Cultures (DSMZ, Braunschweig, Germany). CHO, THP-1, U937 and the 3G8 hybridoma cells were cultured in Roswell Park Memorial Institute (RPMI) 1640 Glutamax-I medium (Invitrogen, Karlsruhe, Germany) containing 10% fetal calf serum (FCS; Invitrogen), 100 units/ml penicillin (Invitrogen), and 100 µg/ml streptomycin (Invitrogen). MOLM-13 were cultured in RPMI 1640 Glutamax-I medium containing 20% FCS, 100 units/ml penicillin and 100 µg/ml streptomycin (Invitrogen). Human 293, 293T (ATCC) and 293 cells stably transfected with CD123 were maintained in DMEM (Invitrogen) Glutamax-I medium containing 10% FCS, 100 units/ml penicillin and 100 µg/ml streptomycin (Invitrogen). The medium for the CD123 expressing cells further contained 400 µg/ml Geneticin (Invitrogen).

### Example 2: Generation and characterization of CD123-specific scFvs

Four new CD123-specific scFv antibody fragments were generated by immunization of BALB/c mice with a recombinant fusion protein consisting of the extracellular part of human CD123 and the Fc portion of a human IgG1. This was performed in accordance with the procedure for generating CD33-specific scFvs (Schwemmlein *et al*, 2006). The Fc portion was used to assure solubility and native conformation of the fusion protein as well as prolonged serum half life due to recycling via the neonatal Fc receptor (FcRn). Spleen RNA of immunized mice was prepared and used to generate a phage display library. This library was panned for CD123-binding phages with intact CD123⁺ cells. The cDNA from reactive phages was subcloned into the prokaryotic expression vector pAK400. Four different clones were expressed in *E*.*coli* and purified from periplasmic extracts under native conditions by affinity chromatography. All four scFVs were strongly enriched with yields between 0.65 and 2.75 mg/l *E*.*coli* culture (Table 1) and reacted in Western blot experiments with antibodies specific for the hexahistidine-tag. All four scFvs bound to CD123⁺ human acute myeloid leukaemia-derived MOLM-13 cells and to CD123-transfected 293 cells, as visualized by flow cytometry. The scFvs failed to react with CD123⁻ 293 cells, indicating that binding was CD123-specific. In calibrated flow cytometry analysis the equilibrium binding constants (K_{D}) of the scFvs were determined and ranged between 4.5 and 101 nM (Table 1). The scFv with the highest affinity, clone 43 (SEQ ID NOs: 1 and 2), was used for the generation of the sctb [123 x ds16 x 33]. Recently a fifth scFv (clone 52; SEQ ID NOs: 9 and 10) was isolated, which is currently characterized.

**Table 1: Expression yields and equilibrium binding constants (K_{D}) of the CD123 scFvs.**

| | scFv clone | | | | |
|---|---|---|---|---|---|
| | 19 | 26 | 43 | 48 | 52* |
| yield (mg/l E.coli culture) | 0.75 | 2.75 | 0.65 | 0.72 | - |
| K_{D} (nM) | 19.8 ± 3.1 | 101 ± 7.1 | 4.5 ± 0.6 | 56.1 ± 3.8 | - |

| | | | | | |
|---|---|---|---|---|---|
| * characterization in progress | | | | | |

### Example 3: Construction, expression and binding characteristics of sctb [123 x ds16 x 33]

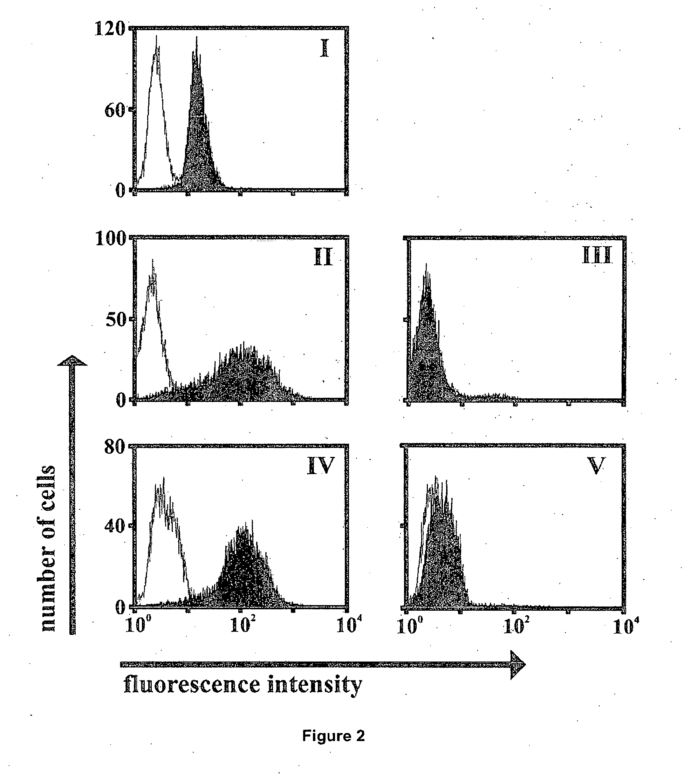

For the construction of the sctb [123 x ds16 x 33] expression vector the already existing vector pSecTag2HygroC-STREP-His-CD33xdsCD16xCD33 was used. To generate the expression vector for the sctb [33 x ds16 x 33], the coding sequence for the CD33-specific scFv was excised from the vector pet27b(+)-Strep-His-CD33-ETA-KDEL (Schwemmlein *et al,* 2006) and cloned as an Sfil cassette into the vector pSecTag2HygroC-STREP-His-dsCD19xdsCD16xdsCD19 (Kellner *et al*, 2008), replacing the coding sequences for the N-terminal dsCD19-specific scFv, and thus generating pSecTag2HygroC-STREP-His-CD33xdsCD16xdsCD19. To produce the vector pSecTag2HygroC-STREP-His-CD33xdsCD16xCD33, the sequence coding for the CD33-specific scFv was amplified by PCR from pet27b(+)-Strep-His-CD33-ETA-KDEL and ligated into pSecTag2HygroC-STREP-His-CD33xdsCD16xdsCD19, using XhoI/EcoRV restriction sites, and replacing the coding sequences for the C-terminal dsCD19-specific scFv. The sequence coding for the CD123-specific scFv was excised from the pAK400-CD123scFv expression construct and cloned as a Sfil-cassette into the vector pSecTag2HygroC-STREP-His-CD33xdsCD16xCD33. Thereby the vector pSecTag2HygroC-STREP-His-CD123xdsCD16xCD33 was produced (comprising the nucleic acid sequence as shown in SEQ ID NO:27). Correct construction of the final constructs was confirmed by DNA sequence analysis on an AppliedBiosystems automated DNA sequencer (ABI Prism 310 Genetic Analyzer; Perkin-Elmer, Ueberlingen, Germany). For expression of the recombinant sctb [123 x ds16 x 33], 293T cells were transfected with the respective expression vector using the calcium phosphate technique including chloroquine. After 10 h, the transfection medium was replaced by fresh culture medium. Supernatants were collected every day for 5 d and combined. Supernatants were analyzed for the presence of antibody fragments by flow cytometry. For expression of the control sctb ds[19 x 16 x 19] specific for the cell surface antigen CD 19, 293T cells were stably transfected with the respective expression vector and cultured under permanent selection with hygromycin C in a mini-PERM bioreactor (Greiner Bio-One, Frickenhausen, Germany) with a dialysis membrane of 12.5 kDa in accordance with the manufacturer's instructions. The medium containing recombinant protein was usually collected 4 times in a period of two weeks. The recombinant His-tagged proteins were enriched by affinity chromatography with nickel-nitrilotriacetic acid agarose (NTA) beads (Qiagen, Hilden, Germany) and finally dialyzed against phosphate-buffered saline (PBS). The average yield of the purified sctb [123 x ds16 x 33] was 0.1 mg per liter of culture medium.
Eluted protein was analyzed by reducing sodium dodecyl sulphate polyacrylamide gel electrophoresis (SDS-PAGE) using standard procedures (Laemmli, 1970). Gels were stained with Coomassie brilliant blue R250 (Sigma-Aldrich, Taufkirchen. Germany). In Western blot experiments, the recombinant protein was detected with an unconjugated penta-His antibody (Qiagen) and a secondary horseradish peroxidase-coupled goat anti-mouse IgG antibody (Dianova, Hamburg Germany). Western Blots were developed using enhanced chemiluminescence reagents (Amersham Pharmacia Biotech, Freiburg, Germany). The electrophoretic mobility of the sctb [123 x ds16 x 33] corresponded to the mass of 89.4 kDa calculated from the sequence (Fig. 1A/B).
Immunofluorescence analysis was performed on a FACSCalibur instrument using CellQuest software (Becton Dickinson, Heidelberg, Germany) as described (Schwemmlein *et al*, 2006). Briefly, 1 x 10⁴ events were collected for each sample, and whole cells were analyzed using appropriate scatter gates to exclude cellular debris and aggregates. The recombinant protein sctb [123 x ds16 x 33] was detected using a penta-His antibody and a PE-conjugated goat anti-mouse-IgG antibody (DAKO Diagnostica GmbH, Hamburg, Germany). Sctb [123 x ds16 x 33] specifically reacted with CD123⁻/CD16⁻/CD33⁺ U937 cells, CD123-transfected CD16⁻/CD33⁻ 293 cells and CD16-transfected CD123⁻/CD33⁻ CHO cells and showed no reaction with untransfected 293 or CHO cells (Fig. 2C). The sctb [123 x ds16 x 33] bound to U937, CD123-transfected 293 and CD16-transfected CHO cells with K_{D}-values of 17.8 ± 2.2, 18.8 ± 0.9 and 21.7 ± 1.8 nM, respectively (Table 2). The avidity of sctb [123 x ds16 x 33] to CD123 and CD33 double positive MOLM-13 cells was 13.5 ± 0.6 nM (Table 2). K_{D} values were determined by calibrated flow cytometry, as described (Benedict *et al,* 1997). The highest mean fluorescence value was set to 100%, and all data points were normalized to this value. The experiments were repeated 6 times. The K_{D} values were calculated using a nonlinear regression curve fit.
The ability of sctb [123 x ds16 x 33] to simultaneously bind all three antigens was tested in flow cytometry analyses. In this experimental setup CD123⁻/CD16⁻/CD33⁺ U937 cells were incubated with sctb [123 x ds16 x 33] and stained in parallel with the recombinant fusion proteins CD123ex-RFP and CD16ex-GFP. These proteins contain the extracellular domain (ex) of the cell surface antigens CD123 and CD16, genetically fused to the red fluorescence protein (RFP) and the green fluorescence protein (GFP), respectively. After extensive washing and removing unbound sctb, CD123ex-RFP and CD16ex-GFP, RFP and GFP double positive cells were detected. This result proved the simultaneous binding of all three scFvs to their respective target antigens.
To determine the *in vitro* serum stability of sctb [123 x ds16 x 33], aliquots of the recombinant protein at a sub-saturating concentration of 2 µg/ml were incubated at 37°C (day 5) in human serum or stored at -20°C and thawed up at defined time points (day 0-4). The residual binding activity was measured by flow cytometry (as described above). The experiment was repeated four times and results were fitted to a monoexponential decay.

**Table 2: Equilibrium binding constants (K_{D}) and stability in human serum at 37°C for 5 days of the scFvs CD123, CD33 and CD16 in the sctb [123 x ds16 x 33].**

| | scFv | | | |
|---|---|---|---|---|
| | CD16 | CD33 | CD123 | CD33/CD123 |
| K_{D}(nM) | 21.7 ± 1.8 | 17.8 ± 2.2 | 18.8 ± 0.9 | 13.5 ± 0.6 |
| stable in human serum at 37°C | yes | yes | yes | yes |

### Example 4: ADCC of established acute myeloid leukaemia-derived cell-lines mediated by sctb [123 x ds16 x 33]

To determine the ability of sctb [123 x ds16 x 33] in mediating ADCC, AML-derived cell-lines were incubated with the sctb and with freshly prepared unstimulated MNCs from unrelated healthy donors (Fig. 4).
For this, citrate buffered peripheral blood from healthy volunteers was obtained after receiving informed consent and with the approval of the Ethics Committee of the University of Erlangen-Nuremberg. MNCs were enriched by Lymphoflot (Biotest, Dreieich, Germany) Ficoll density centrifugation in Leukosep tubes (Greiner, Frickenhausen, Germany) according to manufacturers' instructions, and suspended in RPMI 1640 Glutamax-I medium containing 10% FCS and penicillin and streptomycin at 100 units/mL and 100 mg/mL, respectively. Viability was verified by Trypan blue exclusion and exceeded 95%.
ADCC assays, using MNCs from healthy donors as effector cells, were performed in triplicate using a 3-hour ⁵¹Cr release assay as described (Elsasser *et al,* 1996). Dose-response curves were recorded using several equimolar 5-fold serial dilutions of the respective antibody fragments at a constant effector to target (E:T) cell ratio of 40:1 MNCs to target cells. Background lysis induced by MNCs alone was subtracted from each data point, and EC₅₀ values (concentration of an antibody fragment producing 50% of maximum specific lysis) were calculated by using a sigmoidal dose-response curve fit: The experiments were repeated 4 times and mean values are reported.
In these ADCC reactions the sctb [123 x ds16 x 33] mediated effective tumor cell lysis at low picomolar concentrations with EC₅₀-values of 13 pM for MOLM-13 (Fig. 4A) and 201 pM for THP-1 (Fig. 4B) whereas a control sctb was ineffective. The maximal specific lysis observed for MOLM-13 cells was greater than 50 % and for THP-1 greater than 30 % in a 3 h assay.

### Example 5: Graphical and statistical analysis

Graphical and statistical analyses were performed using Graph Pad Prism Software (Graph Pad Software Inc, San Diego, CA) and Microsoft EXCEL. Group data were reported as means ± standard error of the mean (SEM). Differences between groups were analyzed using unpaired Student *t* test. *P* values < 0.05 were considered significant.

### Example 6: ADCC of primary leukaemia cells from patients mediated by sctbs [123 x ds16 x 33], [123 x ds16 x 123] and [33 x ds16 x 33]

For these experiments, isolated MNCs from either peripheral blood or bone marrow of seven patients were used in ADCC reactions. Overall, six peripheral blood and two bone marrow samples were studied. MNCs from one unrelated healthy donor per AML patient sample were used as effector cells. ADCC assays were performed as described above (Example 4). For direct comparison of the potencies of the dual targeting and mono targeting sctbs in ADCC of primary leukaemia cells, the previously published sctbs [33 x ds16 x 33] (Singer *et al*, 2010) and [123 x ds16 x 123] (Kugler et al. 2010) were also carried along. All three sctbs showed potent lysis of primary AML cells in a concentration-dependent manner (Fig. 5). Four representative ADCC reactions of samples from patients 1 and 2 (peripheral blood; Fig 5A+B) and patients 6 and 7 (bone marrow; Fig 5C+D) are depicted. At a concentration of 5 nM, all three recombinant proteins produced potent specific lysis, however, in 4/8 samples, the dual targeting sctb [123 x ds16 x 33] reached the highest extent of lysis at this particular concentration. This finding was confirmed when ADCC data from all six peripheral blood samples were combined (Fig 5E). The EC₅₀ values derived from this data set were ~250, -130, and -250 pM for the sctbs [123 x ds16 x 33], [123 x ds16 x 123] and [33 x ds16 x 33], respectively. Maximum specific lysis was ~25%, ~19% and ~19% of input cells, respectively.

### References:

Adams, G.P., Schier, R., Marshall, K., Wolf, E.J., McCall, A.M., Marks, J.D. & Weiner, L.M. (1998) Increased affinity leads to improved selective tumor delivery of single-chain Fv antibodies. Cancer Res, 58, 485-490.
Bakker, A.B., van den Oudenrijn, S., Bakker, A.Q., Feller, N., van Meijer, M., Bia, J.A., Jongeneelen, M.A., Visser, T.J., Bijl, N., Geuijen, C.A., Marissen, W.E., Radosevic, K., Throsby, M., Schuurhuis, G.J., Ossenkoppele, G.J., de Kruif, J., Goudsmit, J. & Kruisbeek, A.M. (2004) C-type lectin-like molecule-1: a novel myeloid cell surface marker associated with acute myeloid leukaemia. Cancer Res, 64, 8443-8450.
Bargou, R., Leo, E., Zugmaier, G., Klinger, M., Goebeler, M., Knop, S., Noppeney, R., Viardot, A., Hess, G., Schuler, M., Einsele, H., Brandl, C., Wolf, A., Kirchinger, P., Klappers, P., Schmidt, M., Riethmuller, G., Reinhardt, C., Baeuerle, P.A. & Kufer, P. (2008) Tumor regression in cancer patients by very low doses of a T cell-engaging antibody. Science, 321, 974-977.
Bebbington CR, Renner G, Thomson S, King D, Abrams D, Yarranton GT. High-level expression of a recombinant antibody from myeloma cells using a glutamine synthetase gene as an amplifiable selectable marker. Bio/Technology 1992; 10:169-175.
Benedict, C.A., MacKrell, A.J. & Anderson, W.F. (1997) Determination of the binding affinity of an anti-CD34 single-chain antibody using a novel, flow cytometry based assay. J Immunol Methods, 201,223-231.
Beste, G., Schmidt, F.S., Stibora, T., Skerra, A. (1999) Small antibody-like proteins with prescribed ligand specificities derived from the lipocalin fold. PNAS, 96, 1898-903.
Bonnet, D. & Dick, J.E. (1997) Human acute myeloid leukaemia is organized as a hierarchy that originates from a primitive hematopoietic cell. Nat Med, 3, 730-737.
Braasch DA and Corey DR. Locked nucleic acid (LNA): fine-tuning the recognition of DNA and RNA. Chem Biol. 2001; 8(1):1-7.
Brinkmann U, Lee BK and Pastan I. Recombinant Immunotoxins Containing the VH or VL Domain of Monoclonal Antibody B3 fused to Pseudomonas Exotoxin. J. Immunology. 1993; 150:2774-2782.
Bross, P.F., Beitz, J., Chen, G., Chen, X.H., Duffy, E., Kieffer, L., Roy, S., Sridhara, R., Rahman, A., Williams, G. & Pazdur, R. (2001) Approval summary: gemtuzumab ozogamicin in relapsed acute myeloid leukaemia. Clin Cancer Res, 7, 1490-1496.
Bruenke, J., Fischer, B., Barbin, K., Schreiter, K., Wachter, Y., Mahr, K., Titgemeyer, F., Niederweis, M., Peipp, M., Zunino, S.J., Repp, R., Valerius, T. & Fey, G.H. (2004) A recombinant bispecifc single-chain Fv antibody against HLA class II and FcgammaRIII (CD16) triggers effective lysis of lymphoma cells. Br J Haematol, 125, 167-179.
Bruenke, J., Barbin, K., Kunert, S., Lang, P., Pfeiffer, M., Stieglmaier, K., Niethammer, D., Stockmeyer, B., Peipp, M., Repp, R., Valerius, T. & Fey, G.H. (2005) Effective lysis of lymphoma cells with a stabilised bispecific single-chain Fv antibody against CD19 and FcgammaRIII (CD16). Br J Haematol, 130, 218-228.
Coloma, M.J. & Morrison, S.L. (1997) Design and production of novel tetravalent bispecific antibodies. Nat Biotechnol, 15, 159-163.
Cros, E., Jordheim, L., Dumontet, C. & Galmarini, C.M. (2004) Problems related to resistance to cytarabine in acute myeloid leukaemia. Leuk Lymphoma, 45, 1123-1132.
Daeron, M. (1997) Fc receptor biology. Annu Rev Immunol, 15, 203-234.
de Palazzo IG, Kitson J, Gercel-Taylor C, Adams S, Weiner LM. Bispecific monoclonal antibody regulation of Fc gamma RIII-directed tumor cytotoxicity by large granular lymphocytes. Cell Immunol. 1992;142:338-347.
Dinndorf, P.A., Andrews, R.G., Benjamin, D., Ridgway, D., Wolff, L. & Bernstein, I.D. (1986) Expression of normal myeloid-associated antigens by acute leukaemia cells. Blood, 67, 1048-1053.
Elsasser, D., Valerius, T., Repp, R., Weiner, G.J., Deo, Y., Kalden, J.R., van de Winkel, J.G., Stevenson, G.T., Glennie, M.J. & Gramatzki, M. (1996) HLA class II as potential target antigen on malignant B cells for therapy with bispecific antibodies in combination with granulocyte colony-stimulating factor. Blood, 87, 3803-3812.
Fleit, H.B., Wright, S.D. & Unkeless, J.C. (1982) Human neutrophil Fc gamma receptor distribution and structure. Proc Natl Acad Sci U S A, 79, 3275-3279.
Freeman, S.D., Kelm, S., Barber, E.K. & Crocker, P.R. (1995) Characterization of CD33 as a new member of the sialoadhesin family of cellular interaction molecules. Blood, 85, 2005-2012.
Gardin, C., Turlure, P., Fagot, T., Thomas, X., Terre, C., Contentin, N., Raffoux, E., de Botton, S., Pautas, C., Reman, O., Bourhis, J.H., Fenaux, P., Castaigne, S., Michallet, M., Preudhomme, C., de Revel, T., Bordessoule, D. & Dombret, H. (2007) Postremission treatment of elderly patients with acute myeloid leukaemia in first complete remission after intensive induction chemotherapy: results of the multicenter randomized Acute Leukaemia French Association (ALFA) 9803 trial. Blood, 109; 5129-5135.
Gebauer, M. and Skerra, A. (2009) Engineered protein scaffolds as next-generation antibody therapeutics. Curr Opin Chem Biol, 13, 245-255.
Ghahroudi MA, Desmyter A, Wyns L, Hamers R and Muyldermans S. Selection and identification of single domain antibody fragments from camel heavy-chain antibodies. FEBS Letters 1997; 414:521-526.
Glockshuber R, Malia M, Pfitzinger I, Pluckthun A. A comparison of strategies to stabilize immunoglobulin Fv-fragments. Biochemistry. 1990;29:1362-1367.
Grossbard, M.L., Press, O.W., Appelbaum, F.R., Bernstein, I.D. & Nadler, L.M. (1992) Monoclonal antibody-based therapies of leukaemia and lymphoma. Blood, 80, 863-878.
Hamann, P.R., Hinman, L.M., Hollander, I., Beyer, C.F., Lindh, D., Holcomb, R., Hallett, W., Tsou, H.R., Upeslacis, J., Shochat, D., Mountain, A., Flowers, D.A. & Bernstein, I. (2002) Gemtuzumab ozogamicin, a potent and selective anti-CD33 antibody-calicheamicin conjugate for treatment of acute myeloid leukaemia. Bioconjug Chem, 13, 47-58.
Hartmann F, Renner C, Jung W, Deisting C, Juwana M, Eichentopf B, Kloft M, Pfreundschuh M. Treatment of refractory Hodgkin's disease with an anti-CD16/CD30 bispecific antibody. Blood. 1997;89:2042-2047.
Hauswirth, A.W., Florian, S., Printz, D., Sotlar, K., Krauth, M.T., Fritsch, G., Schernthaner, G.H., Wacheck, V., Selzer, E., Sperr, W.R. & Valent, P. (2007) Expression of the target receptor CD33 in CD34+/CD38-/CD123+ AML stem cells. Eur J Clin Invest, 37, 73-82.
Hombach A, Jung W, Pohl C, Renner C, Sahin U, Schmits R, Wolf J, Kapp U, Diehl V, Pfreundschuh M. A CD16/CD30 bispecific monoclonal antibody induces lysis of Hodgkin's cells by unstimulated natural killer cells in vitro and in vivo. Int J Cancer. 1993;55:830-836.
Hope, K.J., Jin, L. & Dick, J.E. (2004) Acute myeloid leukaemia originates from a hierarchy of leukemic stem cell classes that differ in self-renewal capacity. Nat Immunol, 5, 738-743.
Hosen, N., Park, C.Y., Tatsumi, N., Oji, Y., Sugiyama, H., Gramatzki, M., Krensky, A.M. & Weissman, I.L. (2007) CD96 is a leukemic stem cell-specific marker in human acute myeloid leukaemia. Proc Natl Acad Sci U S A, 104, 11008-11013.
Hu, S., Shively, L., Raubitschek, A., Sherman, M., Williams, L.E., Wong, J.Y., Shively, J.E. & Wu, A.M. (1996) Minibody: A novel engineered anti-carcinoembryonic antigen antibody fragment (single-chain Fv-CH3) which exhibits rapid, high-level targeting of xenografts. Cancer Res, 56, 3055-3061.
Huang, S., Chen, Z., Yu, J.F., Young, D., Bashey, A., Ho, A.D. & Law, P. (1999) Correlation between IL-3 receptor expression and growth potential of human CD34+ hematopoietic cells from different tissues. Stem Cells, 17, 265-272.
Huhalov, A. & Chester, K.A. (2004) Engineered single chain antibody fragments for radioimmunotherapy. Q J Nucl Med Mol Imaging, 48, 279-288.
Huston, J.S., Levinson, D., Mudgett-Hunter, M., Tai, M.S., Novotny, J., Margolies, M.N., Ridge, R.J., Bruccoleri, R.E., Haber, E., Crea, R. & et al. (1988) Protein engineering of antibody binding sites: recovery of specific activity in an anti-digoxin single-chain Fv analogue produced in Escherichia coli. Proc Natl Acad Sci U S A, 85, 5879-5883.
Jedema, I., Barge, R.M., van der Velden, V.H., Nijmeijer, B.A., van Dongen, J.J., Willemze, R. & Falkenburg, J.H. (2004) Internalization and cell cycle-dependent killing of leukemic cells by Gemtuzumab Ozogamicin: rationale for efficacy in CD33-negative malignancies with endocytic capacity. Leukaemia, 18, 316-325.
Jemal, A., Siegel, R., Ward, E., Hao, Y., Xu, J., Murray, T. & Thun, M.J. (2008) Cancer statistics, 2008. CA Cancer J Clin, 58, 71-96.
Jin, L., Hope, K.J., Zhai, Q., Smadja-Joffe, F. & Dick, J.E. (2006) Targeting of CD44 eradicates human acute myeloid leukemic stem cells. Nat Med, 12, 1167-1174.
Jin, L., Lee, E.M., Ramshaw, H.S., Busfield, S.J., Peoppl, A.G., Wilkinson, L., Guthridge, M.A., Thomas, D., Barry, E.F., Boyd, A., Gearing, D.P., Vairo, G., Lopez, A.F., Dick, J.E. & Lock, R.B. (2009) Monoclonal antibody-mediated targeting of CD123, IL-3 receptor alpha chain, eliminates human acute myeloid leukemic stem cells. Cell Stem Cell, 5, 31-42.
Jordan, C.T., Upchurch, D., Szilvassy, S.J., Guzman, M.L., Howard, D.S., Pettigrew, A.L., Meyerrose, T., Rossi, R., Grimes, B., Rizzieri, D.A., Luger, S.M. & Phillips, G.L. (2000) The inteneukin-3 receptor alpha chain is a unique marker for human acute myelogenous leukaemia stem cells. Leukaemia, 14, 1777-1784.
Kabat EA, Wu TT. Identical V region amino acid sequences and segments of sequences in antibodies of different specificities. Relative contributions of VH and VL genes, minigenes, and complementarity-determining regions to binding of antibody-combining sites. J Immunol. 1991;147:1709-1719.
Kellner, C., Bruenke, J., Stieglmaier, J., Schwemmlein, M., Schwenkert, M., Singer, H., Mentz, K., Peipp, M., Lang, P., Oduncu, F., Stockmeyer, B. & Fey, G.H. (2008) A novel CD19-directed recombinant bispecific antibody derivative with enhanced immune effector functions for human leukemic cells. J Immunother, 31,871-884.
Kern, W. & Estey, E.H. (2006) High-dose cytosine arabinoside in the treatment of acute myeloid leukaemia: Review of three randomized trials. Cancer, 107, 116-124.
Kipriyanov, S.M., Cochlovius, B., Schafer, H.J., Moldenhauer, G., Bahre, A., Le Gall, F., Knackmuss, S. & Little, M. (2002) Synergistic antitumor effect of bispecific CD19 x CD3 ahd CD19 x CD16 diabodies in a preclinical model of non-Hodgkin's lymphoma. J Immunol, 169, 137-144.
Kipriyanov, S.M., Moldenhauer, G., Schuhmacher, J., Cochlovius, B., Von der Lieth; C.W., Matys, E.R. & Little, M. (1999) Bispecific tandem diabody for tumor therapy with improved antigen binding and pharmacokinetics. J Mol Biol, 293, 41-56.
Kontermann, R.E. (2005) Recombinant bispecific antibodies for cancer therapy. Acta Pharmacol Sin, 26, 1-9.
Kubetzko, S., Balic, E., Waibel, R., Zangemeister-Wittke, U. & Pluckthun, A. (2006) PEGylation and multimerization of the anti-p185HER-2 single chain Fv fragment 4D5: effects on tumor targeting. J Biol Chem, 281, 35186-35201.
Laemmli UK. Cleavage of structural proteins during the assembly of the head of bacteriophage T4. Nature. 1970; 227(5259): 680-5.
Lapidot, T., Sirard, C., Vormoor, J., Murdoch, B., Hoang, T., Caceres-Cortes, J., Minden, M., Paterson, B., Caligiuri, M.A. & Dick, J.E. (1994) A cell initiating human acute myeloid leukaemia after transplantation into SCID mice. Nature, 367, 645-648.
Larson, R.A., Boogaerts, M., Estey, E., Karanes, C., Stadtmauer, E.A., Sievers, E.L., Mineur, P., Bennett, J.M., Berger, M.S., Eten, C.B., Munteanu, M., Loken, M.R., Van Dongen, J.J., Bernstein, I.D. & Appelbaum, F.R. (2002) Antibody-targeted chemotherapy of older patients with acute myeloid leukaemia in first relapse using Mylotarg (gemtuzumab ozogamicin). Leukaemia, 16, 1627-1636.
Legrand, O., Perrot, J.Y., Baudard, M., Cordier, A., Lautier, R., Simonin, G., Zittoun, R., Casadevall, N. & Marie, J.P. (2000) The immunophenotype of 177 adults with acute myeloid leukaemia: proposal of a prognostic score. Blood, 96, 870-877.
Lu, D., Jimenez, X., Zhang, H., Atkins, A., Brennan, L., Balderes, P., Bohlen, P., Witte, L. & Zhu, Z. (2003) Di-diabody: a novel tetravalent bispecific antibody molecule by design. J Immunol Methods, 279, 219-232.
Mayer, R.J., Davis, R.B., Schiffer, C.A., Berg, D.T., Powell, B.L., Schulman, P., Omura, G.A., Moore, J.O., McIntyre, O.R. & Frei, E., 3rd (1994) Intensive postremission chemotherapy in adults with acute myeloid leukaemia. Cancer and Leukaemia Group B. N Engl J Med, 331, 896-903.
McCall, A.M., Shahied, L., Amoroso, A.R., Horak, E.M., Simmons, H.H., Nielson, U., Adams, G.P., Schier, R.. Marks, J.D. & Weiner, L.M. (2001) Increasing the affinity for tumor antigen enhances bispecific antibody cytotoxicity. J Immunol, 166,6112-6117.
Misaghian, N., Ligresti, G., Steelman, L.S., Bertrand, F.E., Basecke, J., Libra, M., Nicoletti, F., Stivala, F., Milella, M., Tafuri, A., Cervello, M., Martelli, A.M. & McCubrey, J.A. (2009) Targeting the leukemic stem cell: the Holy Grail of leukaemia therapy. Leukaemia, 23, 25-42.
Moretti, S., Lanza, F., Dabusti, M., Tieghi, A., Campioni, D., Dominici, M. & Castoldi, G.L. (2001) CD123 (interleukin 3 receptor alpha chain). J Biol Regul Homeost Agents, 15, 98-100.
Muller, D., Karle, A., Meissburger, B., Hofig, I., Stork, R. & Kontermann, R.E. (2007) Improved pharmacokinetics of recombinant bispecific antibody molecules by fusion to human serum albumin. J Biol Chem, 282, 12650-12660.
Muller, D. & Kontermann, E. (2007) Bispecific Antibodies. In: Handbook of Therapeutic Antibodies (ed. by S. Dübel), Vol. 2, pp. 345 - 378. WILEY-VCH Verlag GMBH & Co. KGaA, Weinheim.
Munoz, L., Nomdedeu, J.F., Lopez, O., Carnicer, M.J., Bellido, M., Aventin, A., Brunet, S. & Sierra, J. (2001) Interleukin-3 receptor alpha chain (CD123) is widely expressed in hematologic malignancies. Haematologica, 86, 1261-1269.
Murphy G, Cockett MI, Ward RV and Docherty AJ. Matrix metalloproteinase degradation of elastin, type IV collagen and proteoglycan. A quantitative comparison of the activities. Biochem J. 1991; 277:277-279.
Nagorsen, D., Bargou, R., Ruttinger, D., Kufer, P., Baeuerle, P.A. & Zugmaier, G. (2009) Immunotherapy of lymphoma and leukaemia with T-cell engaging BiTE antibody blinatumomab. Leuk Lymphoma, 50, 886-891.
Otz, T., Grosse-Hovest, L., Hofmann, M., Rammensee, H.G. & Jung, G. (2009) A bispecific single-chain antibody that mediates target cell-restricted, supra-agonistic CD28 stimulation and killing of lymphoma cells. Leukaemia, 23, 71-77.
Owens GC., Chappell SA., Mauro VP. and Edelman GM. Identification of two short internal ribosome entry sites selected from libraries of random oligonucleotides. Proc. Natl. Acad. Sci. USA 2001; 98(4): 1471-1476.
Peipp, M. & Valerius, T. (2002) Bispecific antibodies targeting cancer cells. Biochem Soc. Trans, 30, 507-511.
Raghavan, M., Chen, M.Y., Gastinel, L.N. & Bjorkman, P.J. (1994) Investigation of the interaction between the class I MHC-related Fc receptor and its immunoglobulin G ligand. Immunity, 1, 303-315.
Ravandi, F. & Estrov, Z. (2006) Eradication of leukaemia stem cells as a new goal of therapy in leukaemia. Clin Cancer Res, 12, 340-344.
Ravetch JV, Kinet JP. Fc receptors. Annu Rev Immunol. 1991;9:457-492.
Reiter, Y., Brinkmann, U., Kreitman, R.J., Jung, S.H., Lee, B. & Pastan, I. (1994) Stabilization of the Fv fragments in recombinant immunotoxins by disulfide bonds engineered into conserved framework regions. Biochemistry, 33, 5451-5459.
Schoonjans, R., Willerns, A., Schoonooghe, S., Fiers, W., Grooten, J. & Mertens, N. (2000) Fab chains as an efficient heterodimerization scaffold for the production of recombinant bispecific and trispecific antibody derivatives. J Immunol, 165, 7050-7057.
Schwemmlein, M., Peipp, M., Barbin, K., Saul, D., Stockmeyer, B., Repp, R., Birkmann, J., Oduncu, F., Emmerich, B. & Fey, G.H. (2006) A CD33-specific single-chain immunotoxin mediates potent apoptosis of cultured human myeloid leukaemia cells. Br J Haematol, 133, 141-151.
Shahied, L.S., Tang, Y., Alpaugh, R.K., Somer, R., Greenspon, D. & Weiner, L.M. (2004) Bispecific minibodies targeting HER2/neu and CD16 exhibit improved tumor lysis when placed in a divalent tumor antigen binding format. J Biol Chem, 279, 53907-53914.
Sievers, E.L. (2001) Efficacy and safety of gemtuzumab ozogamicin in patients with CD33-positive acute myeloid leukaemia in first relapse. Expert Opin Biol Ther, 1, 893-901.
Tang, Y., Lou, J., Alpaugh, R.K., Robinson, M.K., Marks, J.D. & Weiner, L.M. (2007) Regulation of antibody-dependent cellular cytotoxicity by IgG intrinsic and apparent affinity for target antigen. J Immunol, 179, 2815-2823.
Taussig, D.C., Pearce, D.J., Simpson, C., Rohatiner, A.Z., Lister, T.A., Kelly, G., Luongo, J.L., Danet-Desnoyers, G.A. & Bonnet, D. (2005) Hematopoietic stem cells express multiple myeloid markers: implications for the origin and targeted therapy of acute myeloid leukaemia. Blood, 106, 4086-4092.
Tedder, T.F., Baras, A. & Xiu, Y. (2006) Fcgamma receptor-dependent effector mechanisms regulate CD19 and CD20 antibody immunotherapies for B lymphocyte malignancies and autoimmunity. Springer Semin Immunopathol, 28, 351-364.
Testa, U., Riccioni, R., Diverio, D., Rossini, A., Lo Coco, F. & Peschle, C. (2004) Interleukin-3 receptor in acute leukaemia. Leukaemia, 18, 219-226.
Todorovska, A., Roovers, R.C., Dolezal, O., Kortt, A.A., Hoogenboom, H.R. & Hudson, P.J. (2001) Design and application of diabodies, triabodies and tetrabodies for cancer targeting. J Immunol Methods, 248, 47-66.
Uchida, J., Hamaguchi, Y., Oliver, J.A., Ravetch, J.V., Poe, J.C., Haas, K.M. & Tedder, T.F. (2004) The innate mononuclear phagocyte network depletes B lymphocytes through Fc receptor-dependent mechanisms during anti-CD20 antibody immunotherapy. J Exp Med, 199, 1659-1669. van de Winkel JG, Anderson CL. Biology of human immunoglobulin G Fc receptors. J Leukoc Biol. 1991;49:511-524.
von der Lieth, C.W., Bohne-Lang, A., Lohmann, K. K. (2003) Datenbanken und Bioinformatik-Werkzeuge fur die Glykobiologie; BioSpektrum, 5, 636-638
Wang, W.W., Das, D., Tang, X.L.. Budzynski, W. & Suresh, M.R. (2005) Antigen targeting to dendritic cells with bispecific antibodies. J Immunol Methods, 306, 80-92.
Weiner GJ, De Gast GC. Bispecific monoclonal antibody therapy of B-cell malignancy. Leuk Lymphoma. 1995;16:199-207.
Willuda, J., Honegger, A., Waibel, R., Schubiger, P.A., Stahel, R., Zangemeister-Wittke, U & Pluckthun, A. (1999) High thermal stability is essential for tumor targeting of antibody fragments: engineering of a humanized anti-epithelial glycoprotein-2 (epithelial cell adhesion molecule) single-chain Fv fragment. Cancer Res, 59, 5758-5767.

## Claims

1. A molecule having binding specificities for
(a) CD123;
(b) CD16; and
(c) CD33;
wherein the binding specificities are conferred by VH and/or VL domains; or
wherein the binding specificities are conferred by anticalins, adnectins, affibodies or DARPins.

2. The molecule of claim 1, wherein the binding portions of the molecule conferring the specificities to (a), (b) and (c) are polypeptides.

3. The molecule according to any one of claim 1 or 2, which is a single polypeptide chain.

4. The molecule according to any one of claims 1 to 3, wherein the binding portions of the molecule conferring the specificities to (a), (b) and (c) are linked by a linker.

5. The molecule according to any one of claims 1 to 4, wherein the molecule comprises
(d) a first immunoglobulin domain comprising a VL domain linked to a VH domain, wherein the immunoglobulin domain specifically binds to CD123;
(e) a second immunoglobulin domain comprising a VL domain linked to a VH domain, wherein the immunoglobulin domain specifically binds to CD16; and
(f) a third immunoglobulin domain comprising a VL domain linked to a VH domain, wherein the immunoglobulin domain specifically binds to CD33.

6. The molecule of claim 5, wherein at least one immunoglobulin domain comprises at least two cysteine residues capable of forming intramolecular disulfide bridges.

7. The molecule of any one of claims 1 to 6, further comprising at least one additional (poly)peptide.

8. The molecule of any one of claims 5 to 7, wherein the first immunoglobulin domain comprises the amino acid sequence of SEQ ID NO: 2.

9. A nucleic acid molecule encoding the molecule of any one of claims 2 to 8.

10. A vector comprising the nucleic acid molecule of claim 9.

11. A non-human host transformed with the vector of claim 10.

12. A diagnostic composition comprising at least one of
(g) the molecule of any one of claims 1 to 8; or
(h) the nucleic acid molecule of claim 9; or
(i) the vector of claim 10.

13. A pharmaceutical composition comprising at least one of
(j) the molecule of any one of claims 1 to 8; or
(k) the nucleic acid molecule of claim 9; or
(l) the vector of claim 10.

14. The molecule of any one of claims 1 to 8 or the nucleic acid molecule of claim 9 or the vector of claim 10 for use in the treatment of acute myeloid leukaemia and/or myelodysplastic syndrome.

15. The molecule of any one of claims 1 to 8 or the nucleic acid molecule of claim 9 or the vector of claim 10 for use in the treatment of acute myeloid leukaemia and/or myelodysplastic syndrome, wherein the molecule, the nucleic acid molecule, or the vector is to be administered in a remission phase for acute myeloid leukaemia or after diagnosis of myelodysplastic syndrome.

## Patentansprüche

1. Molekül, das Bindespezifitäten für:
(a) CD123;
(b) CD16; und
(c) CD33;
hat;
wobei die Bindespezifitäten von V_{H}- und/oder V_{L}- Domänen vermittelt werden; oder
wobei die Bindespezifitäten von Anticalinen, Adnectinen, Affibodies oder DARPinen vermittelt werden.

2. Molekül nach Anspruch 1, wobei die bindenden Bereiche des Moleküls, die die Spezifitäten für (a), (b), und (c) vermitteln, Polypeptide sind.

3. Molekül nach einem der Ansprüche 1 oder 2, das eine einzelne Polypeptid-Kette ist.

4. Molekül nach einem der Ansprüche 1 bis 3, wobei die bindenden Bereiche des Moleküls, die die Spezifitäten für (a), (b), und (c) vermitteln, durch einen Linker verbunden sind.

5. Molekül nach einem der Ansprüche 1 bis 4, wobei das Molekül umfasst:
(d) eine erste Immunglobulin-Domäne, die eine V_{L}-Domäne umfasst, die mit einer V_{H}-Domäne verknüpft ist, wobei die Immunglobulin-Domäne spezifisch an CD123 bindet;
(e) eine zweite Immunglobulin-Domäne, die eine V_{L}-Domäne umfasst, die mit einer V_{H}-Domäne verknüpft ist, wobei die Immunglobulin-Domäne spezifisch an CD16 bindet; und
(f) eine dritte Immunglobulin-Domäne, die eine V_{L}-Domäne umfasst, die mit einer V_{H}-Domäne verknüpft ist, wobei die Immunglobulin-Domäne spezifisch an CD33 bindet.

6. Molekül nach Anspruch 5, wobei mindestens eine Immunglobulin-Domäne mindestens zwei Cystein-Reste umfasst, die intramolekulare Disulfidbrücken ausbilden können.

7. Molekül nach einem der Ansprüche 1 bis 6, das des Weiteren mindestens ein zusätzliches (Poly)peptid umfasst.

8. Molekül nach einem der Ansprüche 5 bis 7, wobei die erste Immunglobulin-Domäne die Aminosäuresequenz der SEQ ID Nr:2 umfasst.

9. Nukleinsäuremolekül, das das Molekül nach einem der Ansprüche 2 bis 8 kodiert.

10. Vektor umfassend das Nukleinsäuremolekül nach Anspruch 9.

11. Nicht-menschlicher Wirt, der mit dem Vektor nach Anspruch 10 transformiert ist.

12. Diagnostische Zusammensetzung umfassend mindestens eines von
(g) dem Molekül nach einem der Ansprüche 1 bis 8; oder
(h) dem Nukleinsäuremolekül nach Anspruch 9; oder
(i) dem Vektor nach Anspruch 10.

13. Pharmazeutische Zusammensetzung umfassend mindestens eines von
(g) dem Molekül nach einem der Ansprüche 1 bis 8; oder
(h) dem Nukleinsäuremolekül nach Anspruch 9; oder
(i) dem Vektor nach Anspruch 10.

14. Molekül nach einem der Ansprüche 1 bis 8, oder Nukleinsäuremolekül nach Anspruch 9, oder Vektor nach Anspruch 10 zur Verwendung in der Behandlung akuter myeloischer Leukämie und/oder des Myelodysplastischen Syndroms.

15. Molekül nach einem der Ansprüche 1 bis 8, oder Nukleinsäuremolekül nach Anspruch 9, oder Vektor nach Anspruch 10 zur Verwendung in der Behandlung akuter myeloischer Leukämie und/oder des Myelodysplastischen Syndroms, wobei das Molekül, das Nukleinsäuremolekül oder der Vektor in einer Remissionsphase bei akuter myeloischer Leukämie oder nach der Diagnose des Myelodysplastischen Syndroms zu verabreichen ist.

## Revendications

1. Molécule ayant des spécificités de liaison pour
(a) CD 123 ;
(b) CD16 ; et
(c) CD33 ;
dans laquelle les spécificités de liaison sont conférées par les domaines VH et/ou VL ; ou
dans laquelle les spécificités de liaison sont conférées par les anticalines, les adnectines, les afficorps ou les DARPines.

2. Molécule selon la revendication 1, dans laquelle les parties liantes de la molécule conférant les spécificités (a), (b) et (c) sont des polypeptides.

3. Molécule selon l'une quelconque des revendications 1 ou 2, qui est une chaîne polypeptidique unique.

4. Molécule selon l'une quelconque des revendications 1 à 3, dans laquelle les parties liantes de la molécule conférant les spécificités (a), (b) et (c) sont liées par un lieur.

5. Molécule selon l'une quelconque des revendications 1 à 4, dans laquelle la molécule comprend
(d) un premier domaine d'immunoglobuline comprenant un domaine VL lié à un domaine VH, le domaine d'immunoglobuline se liant spécifiquement à CD123 ;
(e) un deuxième domaine d'immunoglobuline comprenant un domaine VL lié à un domaine VH, le domaine d'immunoglobuline se liant spécifiquement à CD16 ; et
(f) un troisième domaine d'immunoglobuline comprenant un domaine VL lié à un domaine VH, le domaine d'immunoglobuline se liant spécifiquement à CD33.

6. Molécule selon la revendication 5, dans laquelle au moins un domaine d'immunoglobuline comprend au moins deux résidus cystéine capables de former des ponts disulfure intramoléculaires.

7. Molécule selon l'une quelconque des revendications 1 à 6, comprenant en outre au moins un (poly)peptide supplémentaire.

8. Molécule selon l'une quelconque des revendications 5 à 7, dans laquelle le premier domaine d'immunoglobuline comprend la séquence d'acides aminés de SEQ ID N° 2.

9. Molécule d'acide nucléique codant pour la molécule selon l'une quelconque des revendications 2 à 8.

10. Vecteur comprenant la molécule d'acide nucléique selon la revendication 9.

11. Hôte non humain transformé avec le vecteur selon la revendication 10.

12. Composition de diagnostic comprenant au moins l'un
(g) de la molécule selon l'une quelconque des revendications 1 à 8 ; ou
(h) de la molécule d'acide nucléique selon la revendication 9 ; ou
(i) du vecteur selon la revendication 10.

13. Composition pharmaceutique comprenant au moins l'un
(g) de la molécule selon l'une quelconque des revendications 1 à 8 ; ou
(h) de la molécule d'acide nucléique selon la revendication 9 ; ou
(i) du vecteur selon la revendication 10.

14. Molécule selon l'une quelconque des revendications 1 à 8 ou molécule d'acide nucléique selon la revendication 9 ou vecteur selon la revendication 10 pour une utilisation dans le traitement de la leucémie myéloïde aiguë et/ou du syndrome myélodysplasique.

15. Molécule selon l'une quelconque des revendications 1 à 8 ou molécule d'acide nucléique selon la revendication 9 ou vecteur selon la revendication 10 pour une utilisation dans le traitement de la leucémie myéloïde aiguë et/ou du syndrome myélodysplasique, dans laquelle la molécule, la molécule d'acide nucléique ou le vecteur est destiné à être administré dans une phase de rémission de la leucémie myéloïde aiguë ou après le diagnostic du syndrome myélodysplasique.


### SEQUENCE LISTING

<110> Friedrich-Alexander-Universitat Erlangen-Nürnberg et al.
<120> Therapeutics against acute myeloid leukaemia
<130> R3150 PCT
<150> EP 09 01 5260.4
   <151> 2009-12-09
<160> 28
<170> PatentIn version 3.4
<210> 1
   <211> 780
   <212> DNA
   <213> artificial sequence
<220>
   <223> /note="Description of artificial sequence: CD123 scFv Klon 43"
<400> 1
   atggcggact acaaagatat tgtgatgacc cagtctcaca aattcatgtc cacatcagta      60
   ggagacaggg tcaacatcac ctgcaaggcc agtcagaatg tggatagtgc tgtagcctgg     120
   tatcaacaga aaccagggca atctcctaaa gcactgattt actcggcatc ctaccggtac     180
   agtggagtcc ctgatcgctt cacaggcagg ggatctggga cagatttcac tctcaccatc     240
   agcagtgtgc aggctgaaga cctggcagtt tattactgtc agcaatatta tagcactccg     300
   tggacgttcg gtggaggcac caagctggaa atcaaacgtg gtggtggtgg ttctggtggt     360
   ggtggttctg gcggcggcgg ctccggtggt ggtggatccg aggtgaagct ggtggagtct     420
   ggaggaggct tggtacagcc tgggggttct ctgagtctct cctgtgcagc ttctggattc     480
   accttcactg attactacat gagctgggtc cgccagcctc cagggaaggc acttgagtgg     540
   ttggctttga ttagaagcaa agctgatggt tacacaacag aatacagtgc atctgtgaag     600
   ggtcggttca ccctctccag agatgattcc caaagcatcc tctatcttca aatgaatgcc     660
   ctgagacctg aagacagtgc cacttattac tgtgcaagag atgcggccta ctatagttac     720
   tatagtcccg agggggctat ggactactgg ggtcaaggaa cctcagtcac cgtctcctcg     780
<210> 2
   <211> 260
   <212> PRT
   <213> artificial sequence
<220>
   <223> /note="Description of artificial sequence: CD123 scFv Klon 43"
<400> 2
   Met Ala Asp Tyr Lys Asp Ile Val Met Thr Gln Ser His Lys Phe Met
   1               5                   10                  15
   Ser Thr Ser Val Gly Asp Arg Val Asn Ile Thr Cys Lys Ala Ser Gln
               20                  25                  30
   Asn Val Asp Ser Ala Val Ala Trp Tyr Gln Gln Lys Pro Gly Gln Ser
           35                  40                  45
   Pro Lys Ala Leu Ile Tyr Ser Ala Ser Tyr Arg Tyr Ser Gly Val Pro
       50                  55                  60
   Asp Arg Phe Thr Gly Arg Gly Ser Gly Thr Asp Phe Thr Leu Thr Ile
   65                  70                  75                  80
   Ser Ser Val Gln Ala Glu Asp Leu Ala Val Tyr Tyr Cys Gln Gln Tyr
                   85                  90                  95
   Tyr Ser Thr Pro Trp Thr Phe Gly Gly Gly Thr Lys Leu Glu Ile Lys
               100                 105                 110
   Arg Gly Gly Gly Gly Ser Gly Gly Gly Gly Ser Gly Gly Gly Gly Ser
           115                 120                 125
   Gly Gly Gly Gly Ser Glu Val Lys Leu Val Glu Ser Gly Gly Gly Leu
       130                 135                 140
   Val Gln Pro Gly Gly Ser Leu Ser Leu Ser Cys Ala Ala Ser Gly Phe
   145                 150                 155                 160
   Thr Phe Thr Asp Tyr Tyr Met Ser Trp Val Arg Gln Pro Pro Gly Lys
                   165                 170                 175
   Ala Leu Glu Trp Leu Ala Leu Ile Arg Ser Lys Ala Asp Gly Tyr Thr
               180                 185                 190
   Thr Glu Tyr Ser Ala Ser Val Lys Gly Arg Phe Thr Leu Ser Arg Asp
           195                 200                 205
   Asp Ser Gln Ser Ile Leu Tyr Leu Gln Met Asn Ala Leu Arg Pro Glu
       210                 215                 220
   Asp Ser Ala Thr Tyr Tyr Cys Ala Arg Asp Ala Ala Tyr Tyr Ser Tyr
   225                 230                 235                 240
   Tyr Ser Pro Glu Gly Ala Met Asp Tyr Trp Gly Gln Gly Thr Ser Val
                   245                 250                 255
   Thr Val Ser Ser
               260
<210> 3
   <211> 777
   <212> DNA
   <213> artificial sequence
<220>
   <223> /note="Description of artificial sequence: CD123 scFv Klon 19"
<400> 3
   atggcggact acaaagacat tgtgatgacc caaactccac cctccctgcc tgtcagtctt    60
   ggagatcaag cctccatctc ttgcagatct agtcagagcc ttgtacacag taatggaaac   120
   acctatttac attggtacct gcagaagcca ggccagtctc caaagctcct gatctacaaa   180
   gtttccaacc gattttctgg ggtcccagac aggttcagtg gcagtggatc agggacagat   240
   ttcacactca agatcagcag agtggaggct gaggatctgg gagtttatta ctgctttcaa   300
   ggttcacatg ttcctcggac gttcggtgga ggcaccaagc tggaaataaa acgtggtggt   360
   ggtggttctg gtggtggtgg ttctggcggc ggcggctcca gtggtggtgg atcccaggtg   420
   cagctgcagc agtctggggc tgaactggca aaacctgggg cctcagtgaa actgtcctgc   480
   aaggcttctg gctacacctt tattagctac tggatacact gggtaaaaca gaggcctgga   540
   cagggtctgg aatggattgg atacattaat cctagccgtg gttatactaa ctccaatcag   600
   aagttcaatg acaaggccac attgactgca gacgaatcct ccagtacagc ctacatgcag   660
   ctgagcagcc tgacatatga ggactctgca gtctattact gtgcaagagg ttatgattat   720
   cccgggggaa tgatcattga ctactggggt caaggaacct cagtcaccgt ctcctcg      777
<210> 4
   <211> 259
   <212> PRT
   <213> artificial sequence
<220>
   <223> /note="Description of artificial sequence: CD123 scFv Klon 19"
<400> 4
   Met Ala Asp Tyr Lys Asp Ile Val Met Thr Gln Thr Pro Pro Ser Leu
   1               5                   10                  15
   Pro Val Ser Leu Gly Asp Gln Ala Ser Ile Ser Cys Arg Ser Ser Gln
               20                  25                  30
   Ser Leu Val His Ser Asn Gly Asn Thr Tyr Leu His Trp Tyr Leu Gln
           35                  40                  45
   Lys Pro Gly Gln Ser Pro Lys Leu Leu Ile Tyr Lys Val Ser Asn Arg
       50                  55                  60
   Phe Ser Gly Val Pro Asp Arg Phe Ser Gly Ser Gly Ser Gly Thr Asp
   65                  70                  75                  80
   Phe Thr Leu Lys Ile Ser Arg Val Glu Ala Glu Asp Leu Gly Val Tyr
   85 90 95
   Tyr Cys Phe Gln Gly Ser His Val Pro Arg Thr Phe Gly Gly Gly Thr
   100 105 110
   Lys Leu Glu Ile Lys Arg Gly Gly Gly Gly Ser Gly Gly Gly Gly Ser
   115 120 125
   Gly Gly Gly Gly Ser Ser Gly Gly Gly Ser Gln Val Gln Leu Gln Gln
   130 135 140
   Ser Gly Ala Glu Leu Ala Lys Pro Gly Ala Ser Val Lys Leu Ser Cys
   145 150 155 160
   Lys Ala Ser Gly Tyr Thr Phe Ile Ser Tyr Trp Ile His Trp Val Lys
   165 170 175
   Gln Arg Pro Gly Gln Gly Leu Glu Trp Ile Gly Tyr Ile Asn Pro Ser
   180 185 190
   Arg Gly Tyr Thr Asn Ser Asn Gln Lys Phe Asn Asp Lys Ala Thr Leu
   195 200 205
   Thr Ala Asp Glu Ser Ser Ser Thr Ala Tyr Met Gln Leu Ser Ser Leu
   210 215 220
   Thr Tyr Glu Asp Ser Ala Val Tyr Tyr Cys Ala Arg Gly Tyr Asp Tyr
   225 230 235 240
   Pro Gly Gly Met Ile Ile Asp Tyr Trp Gly Gln Gly Thr Ser Val Thr
   245 250 255
   Val Ser Ser
<210> 5
   <211> 750
   <212> DNA
   <213> artificial sequence
<220>
   <223> /note="Description of artificial sequence: CD123 scFv Klon 26"
<400> 5
   atggcggact acaaagatat tgttatgacc cagtctcaca aattcatgtc cacatcagaa     60
   ggagacaggg tcagtatcac ctgcaaggcc agtcagaacg tggattctgc tgtagcctgg    120
   tatcaacaga aaccagggca atctcctaaa ctactgattt actgggcatc cacccggcac    180
   actggagtcc ctgatcgctt cacaggcagt ggatctggga cggatttcac tctcaccatt    240
   agcaatgtgc agtctgaaga cttggcagat tatttctgtc agcaatatag cagctatcca    300
   ttcacgttcg gctcggggac aaagttggaa ataaaacgtg gtggtggtgg ttctggtggt    360
   ggtggttccg gcggcggcgg ctccggtggt ggtggatccc aggttcaact gcagcagtct    420
   ggggcagaac ttgtgaggcc aggggcctca gtcaagttgt cctgcacaac ttctggcttc    480
   aacattaaag acagctatat tcactgggtg aaacagaggc ctgagcaggg cctggaatgg    540
   attggaagga ttgatcctga agatggtgat actgaatatg ccccgaagtt ccagggcagg    600
   gccactgtga ctgcagacac atcctccaac acagcctact tacaactcag cagcctgaca    660
   tctgaggacg ctgccgtcta ttactgtgct gcaactggga cgggattctt tgacttctgg    720
   ggccaaggca ccactctcac agtctcctcg                                     750
<210> 6
   <211> 250
   <212> PRT
   <213> artificial sequence
<220>
   <223> /note="Description of artificial sequence: CD123 scfv Klon 26"
<400> 6
   Met Ala Asp Tyr Lys Asp Ile Val Met Thr Gln Ser His Lys Phe Met
   1               5                   10                  15
   Ser Thr Ser Glu Gly Asp Arg Val Ser Ile Thr Cys Lys Ala Ser Gln
               20                  25                  30
   Asn Val Asp Ser Ala Val Ala Trp Tyr Gln Gln Lys Pro Gly Gln Ser
           35                  40                  45
   Pro Lys Leu Leu Ile Tyr Trp Ala Ser Thr Arg His Thr Gly Val Pro
       50                  55                  60
   Asp Arg Phe Thr Gly Ser Gly Ser Gly Thr Asp Phe Thr Leu Thr Ile
   65                  70                  75                  80
   Ser Asn Val Gln Ser Glu Asp Leu Ala Asp Tyr Phe Cys Gln Gln Tyr
                   85                  90                  95
   Ser Ser Tyr Pro Phe Thr Phe Gly Ser Gly Thr Lys Leu Glu Ile Lys
               100                 105                 110
   Arg Gly Gly Gly Gly Ser Gly Gly Gly Gly Ser Gly Gly Gly Gly Ser
           115                 120                 125
   Gly Gly Gly Gly Ser Gln Val Gln Leu Gln Gln Ser Gly Ala Glu Leu
       130                 135                 140
   Val Arg Pro Gly Ala Ser Val Lys Leu Ser Cys Thr Thr Ser Gly Phe
   145                 150                 155                 160
   Asn Ile Lys Asp Ser Tyr Ile His Trp Val Lys Gln Arg Pro Glu Gln
                   165                 170                 175
   Gly Leu Glu Trp Ile Gly Arg Ile Asp Pro Glu Asp Gly Asp Thr Glu
               180                 185                 190
   Tyr Ala Pro Lys Phe Gln Gly Arg Ala Thr Val Thr Ala Asp Thr Ser
           195                 200                 205
   Ser Asn Thr Ala Tyr Leu Gln Leu Ser Ser Leu Thr Ser Glu Asp Ala
       210                 215                 220
   Ala Val Tyr Tyr Cys Ala Ala Thr Gly Thr Gly Phe Phe Asp Phe Trp
   225                 230                 235                 240
   Gly Gln Gly Thr Thr Leu Thr Val Ser Ser
                   245                 250
<210> 7
   <211> 780
   <212> DNA
   <213> artificial sequence
<220>
   <223> /note="Description of artificial sequence: CD123 scFv Klon 48"
<400> 7
   atggcggact acaaagatat tgtgatgacc cagtctcaaa aattcatgtc cacatcagta      60
   ggagacaggg tcagcatcac ctgcaaggcc agtcaggatg tgggtactgc tgtagcctgg     120
   tatcaacaga aaccagggca gtctcctaaa gcactgattt acttggcatc caaccggctc     180
   actggagtcc cagatcgctt cacaggcagt ggatctggga cagatttcac tctcaccatc     240
   agcaatgtgc agtctgaaga cttggcagat tatttctgcc agcaatatgg cagctatccg     300
   ctcacgttcg gtgctgggac caagctggaa atcaaacgtg gtggtggtgg ttctggtggt     360
   ggtggttctg gcggcggcgg ctccggtggt ggtggatccg aggtgcaact ggtggagtct     420
   ggaggaggct tggtacagcc tgggggttct ctgagtctct cctgtgcagc ttctggattc     480
   accttcactg attactacat gagctgggtc cgccagcctc cagggaaggc acttgagtgg     540
   ttggctttga ttagaagcaa agctgatggt tacacaacag aatacagtgc atctgtgaag     600
   ggtcggttca ccctctccag agatgattcc caaagcatcc tctatcttca aatgaatgcc     660
   ctgagacctg aagacagtgc cacttattac tgtgcaagag atgcggccta ctatagttac     720
   tatagtcccg agggggctat ggactactgg ggtcaaggaa tctcagtcac cgtctcctcg    780
<210> 8
   <211> 260
   <212> PRT
   <213> artificial sequence
<220>
   <223> /note="Description of artificial sequence: CD123 scFv Klon 48"
<400> 8
   Met Ala Asp Tyr Lys Asp Ile Val Met Thr Gln Ser Gln Lys Phe Met
   1               5                   10                  15
   Ser Thr Ser Val Gly Asp Arg Val Ser Ile Thr Cys Lys Ala Ser Gln
               20                  25                  30
   Asp Val Gly Thr Ala Val Ala Trp Tyr Gln Gln Lys Pro Gly Gln Ser
           35                  40                  45
   Pro Lys Ala Leu Ile Tyr Leu Ala Ser Asn Arg Leu Thr Gly Val Pro
       50                  55                  60
   Asp Arg Phe Thr Gly Ser Gly Ser Gly Thr Asp Phe Thr Leu Thr Ile
   65                  70                  75                  80
   Ser Asn Val Gln Ser Glu Asp Leu Ala Asp Tyr Phe Cys Gln Gln Tyr
                   85                  90                  95
   Gly Ser Tyr Pro Leu Thr Phe Gly Ala Gly Thr Lys Leu Glu Ile Lys
               100                 105                 110
   Arg Gly Gly Gly Gly Ser Gly Gly Gly Gly Ser Gly Gly Gly Gly Ser
           115                 120                 125
   Gly Gly Gly Gly Ser Glu Val Gln Leu Val Glu Ser Gly Gly Gly Leu
       130                 135                 140
   Val Gln Pro Gly Gly Ser Leu Ser Leu Ser Cys Ala Ala Ser Gly Phe
   145                 150                 155                 160
   Thr Phe Thr Asp Tyr Tyr Met Ser Trp Val Arg Gln Pro Pro Gly Lys
                   165                 170                 175
   Ala Leu Glu Trp Leu Ala Leu Ile Arg Ser Lys Ala Asp Gly Tyr Thr
               180                 185                 190
   Thr Glu Tyr Ser Ala Ser Val Lys Gly Arg Phe Thr Leu Ser Arg Asp
           195                 200                 205
   Asp Ser Gln Ser Ile Leu Tyr Leu Gln Met Asn Ala Leu Arg Pro Glu
       210                 215                 220
   Asp Ser Ala Thr Tyr Tyr Cys Ala Arg Asp Ala Ala Tyr Tyr Ser Tyr
   225 230 235 240
   Tyr Ser Pro Glu Gly Ala Met Asp Tyr Trp Gly Gln Gly Ile Ser Val
   245 250 255
   Thr Val Ser Ser
   260
<210> 9
   <211> 750
   <212> DNA
   <213> artificial sequence
<220>
   <223> /note="Description of artificial sequence: CD123 scFv Klon 52"
<400> 9
   atggcggact acaaagatat tgtgatgaca cagactacat cctccctgtc tgcctctctg 60
   ggagacagag tcaccatcag ttgcagggca agtcaggaca ttagcaatta tttaaactgg 120
   tatcagcaga aaccaggaca gccacccaaa ctcctcatct atcttgcatc caacctagaa 180
   tctggggtcc ctgccaggtt cagtggcagt gggtctagga cagacttcac cctcaccatt 240
   gatcctgtgg aggctgatga tgctgcaacc tattactgtc agcaaaataa tgaggatcct 300
   ctcacgttcg gtgctgggac caagctggag ctgaaacgtg gcggcggcgg ctccggtggt 360
   ggtggatccg aggtgaagct gatggagtct ggaggaggct tggtacagcc tgggggttct 420
   ctgagtctct cctgtgcagc ttctggattc accttcactg attactacat gagctgggtc 480
   cgccagcctc cagggaaggc acttgagtgg ttggctttga ttagaagcaa agctgatggt 540
   tacacaacag aatacagtgc atctgtgaag ggtcggttca ccctctccag agatgattcc 600
   caaagcatcc tctatcttca aatgaatgcc ctgagacctg aagacagtgc cacttattac 660
   tgtgcaagag atgcggccta ctatagttac tatagtcccg agggggctat ggactactgg 720
   ggtcaaggaa cctcagtcac cgtctcctcg 750
<210> 10
   <211> 250
   <212> PRT
   <213> artificial sequence
<220>
   <223> /note="Description of artificial sequence: CD123 scFv Klon 52"
<400> 10
   Met Ala Asp Tyr Lys Asp Ile Val Met Thr Gln Thr Thr Ser Ser Leu
   1 5 10 15
   Ser Ala Ser Leu Gly Asp Arg Val Thr Ile Ser Cys Arg Ala Ser Gln
   20 25 30
   Asp Ile Ser Asn Tyr Leu Asn Trp Tyr Gln Gln Lys Pro Gly Gln Pro
   35 40 45
   Pro Lys Leu Leu Ile Tyr Leu Ala Ser Asn Leu Glu Ser Gly Val Pro
   50 55 60
   Ala Arg Phe Ser Gly Ser Gly Ser Arg Thr Asp Phe Thr Leu Thr Ile
   65 70 75 80
   Asp Pro Val Glu Ala Asp Asp Ala Ala Thr Tyr Tyr Cys Gln Gln Asn
   85 90 95
   Asn Glu Asp Pro Leu Thr Phe Gly Ala Gly Thr Lys Leu Glu Leu Lys
   100 105 110
   Arg Gly Gly Gly Gly Ser Gly Gly Gly Gly Ser Glu Val Lys Leu Met
   115 120 125
   Glu Ser Gly Gly Gly Leu Val Gln Pro Gly Gly Ser Leu Ser Leu Ser
   130 135 140
   Cys Ala Ala Ser Gly Phe Thr Phe Thr Asp Tyr Tyr Met Ser Trp Val
   145 150 155 160
   Arg Gln Pro Pro Gly Lys Ala Leu Glu Trp Leu Ala Leu Ile Arg Ser
   165 170 175
   Lys Ala Asp Gly Tyr Thr Thr Glu Tyr Ser Ala Ser Val Lys Gly Arg
   180 185 190
   Phe Thr Leu Ser Arg Asp Asp Ser Gln Ser Ile Leu Tyr Leu Gln Met
   195 200 205
   Asn Ala Leu Arg Pro Glu Asp Ser Ala Thr Tyr Tyr Cys Ala Arg Asp
   210 215 220
   Ala Ala Tyr Tyr Ser Tyr Tyr Ser Pro Glu Gly Ala Met Asp Tyr Trp
   225 230 235 240
   Gly Gln Gly Thr Ser Val Thr Val Ser Ser
   245 250
<210> 11
   <211> 753
   <212> DNA
   <213> artificial sequence
<220>
   <223> /note="Description of artificial sequence: CD123 scFv Klon 42"
<400> 11
   atggcggact acaaagacat tgtgatgaca cagtctcaca aattcatgtc cacatcagta      60
   ggagacaggg tcagcatcac ctgcaaggcc agtcaggatg tgagtactgc tgttgcctgg     120
   tatcaacaga aaccagggca atctcctaaa ctactgattt actgggcatc cacccggcac     180
   actggagtcc ctgatcgctt cacaggcagt ggatctggga cagatttcac tctcaccatt     240
   agcaatgtgc agtctgaaga cttggcagat tatttctgtc agcaatatag cagctatccg     300
   tggacgttcg gtggaggcac caagctggaa ataaaacgcg gtggtggtgg ttctggtggt     360
   ggtggttctg gcggcggcgg ctccggtggt ggtggatccg aggtgcagct acttgagtct     420
   gggggaacct tagtggaccc tggagggtcc ctgaaactct cctgtgcagc ctctggattc     480
   actttcaata actatcccat gtcttgggtt cgccagggtc cggaaaagag gctggagtgg     540
   gtcgccacca ttagttatgg tggtaattac accttctatc cagacaatct aaagggccga     600
   ttcaccatct ccagagacaa tgccaagaac aacctgtatc tgcaaatgag tcatctgaag     660
   tctgcggaca cagccatata ttactgtgcc agacattttg attacgactt ctttgacttc     720
   tggggccaag gcaccactgt caccgtctcc tcg                                  753
<210> 12
   <211> 251
   <212> PRT
   <213> artificial sequence
<220>
   <223> /note="Description of artificial sequence: CD123 scFv Klon 42"
<400> 12
   Met Ala Asp Tyr Lys Asp Ile Val Met Thr Gln Ser His Lys Phe Met
   1               5                   10                  15
   Ser Thr Ser Val Gly Asp Arg Val Ser Ile Thr Cys Lys Ala Ser Gln
               20                  25                  30
   Asp Val Ser Thr Ala Val Ala Trp Tyr Gln Gln Lys Pro Gly Gln Ser
           35                  40                  45
   Pro Lys Leu Leu Ile Tyr Trp Ala Ser Thr Arg His Thr Gly Val Pro
       50                  55                  60
   Asp Arg Phe Thr Gly Ser Gly Ser Gly Thr Asp Phe Thr Leu Thr Ile
   65                  70                  75                  80
   Ser Asn Val Gln Ser Glu Asp Leu Ala Asp Tyr Phe Cys Gln Gln Tyr
                   85                  90                  95
   Ser Ser Tyr Pro Trp Thr Phe Gly Gly Gly Thr Lys Leu Glu Ile Lys
               100                 105                 110
   Arg Gly Gly Gly Gly Ser Gly Gly Gly Gly Ser Gly Gly Gly Gly Ser
           115                 120                 125
   Gly Gly Gly Gly Ser Glu Val Gln Leu Leu Glu Ser Gly Gly Thr Leu
       130                 135                 140
   Val Asp Pro Gly Gly Ser Leu Lys Leu Ser Cys Ala Ala Ser Gly Phe
   145                 150                 155                 160
   Thr Phe Asn Asn Tyr Pro Met Ser Trp Val Arg Gln Gly Pro Glu Lys
                   165                 170                 175
   Arg Leu Glu Trp Val Ala Thr Ile Ser Tyr Gly Gly Asn Tyr Thr Phe
               180                 185                 190
   Tyr Pro Asp Asn Leu Lys Gly Arg Phe Thr Ile Ser Arg Asp Asn Ala
           195                 200                 205
   Lys Asn Asn Leu Tyr Leu Gln Met Ser His Leu Lys Ser Ala Asp Thr
       210                 215                 220
   Ala Ile Tyr Tyr Cys Ala Arg His Phe Asp Tyr Asp Phe Phe Asp Phe
   225                 230                 235                 240
   Trp Gly Gln Gly Thr Thr Val Thr Val Ser Ser
                   245                 250
<210> 13
   <211> 765
   <212> DNA
   <213> artificial sequence
<220>
   <223> /note="Description of artificial sequence: CD123 scFv Klon 45"
<400> 13
   atggcggact acaaagacat ccagatgaca cagtctcctg cttccttagc tgtatctctg     60
   gggcagaggg ccaccatctc atgcagggcc agcaaaagtg tcagtacatc tggctatagt    120
   tatatgcact ggtaccaaca gaaaccagga cagccaccca aactcctcat ctatcttgca    180
   tccaacctag aatctggggt ccctgccagg ttcagtggca gtgggtctgg gacagacttc    240
   accctcaaca tccatcctgt ggaggaggag gatgctgcaa cctattactg tcagcacagt    300
   agggagcttc cgtacacgtt cggagggggg accaagctgg aaatcaaacg cggtggtggt    360
   ggttctggtg gtggtggttc tggcggcggc ggctccggtg gtggtggatc cgaggtgcag    420
   ctggtggagt ctgggggaac cttagtgaag cctggagggt ccctgaaact ctcctgtgca    480
   gcctctggat tcactttcaa taactatccc atgtcttggg ttcgccaggg tccggaaaag    540
   aggctggagt gggtcgccac cattagttat ggtggtaatt acaccttcta tccagacaat    600
   ctaaagggcc gattcaccat ctccagagac aatgccaaga acaacctgta cctgcaaatg    660
   agccatctga agtctgcgga cacagccata tattactgtg ccagacattt tgattacgac    720
   ttctttgact tctggggcac agggaccact ctcacagtct cctcg                    765
<210> 14
   <211> 255
   <212> PRT
   <213> artificial sequence
<220>
   <223> /note="Description of artificial sequence: CD123 scFv Klon 45"
<400> 14
   Met Ala Asp Tyr Lys Asp Ile Gln Met Thr Gln Ser Pro Ala Ser Leu
   1               5                   10                  15
   Ala Val Ser Leu Gly Gln Arg Ala Thr Ile Ser Cys Arg Ala Ser Lys
               20                  25                  30
   Ser Val Ser Thr Ser Gly Tyr Ser Tyr Met His Trp Tyr Gln Gln Lys
           35                  40                  45
   Pro Gly Gln Pro Pro Lys Leu Leu Ile Tyr Leu Ala Ser Asn Leu Glu
       50                  55                  60
   Ser Gly Val Pro Ala Arg Phe Ser Gly Ser Gly Ser Gly Thr Asp Phe
   65                  70                  75                  80
   Thr Leu Asn Ile His Pro Val Glu Glu Glu Asp Ala Ala Thr Tyr Tyr
                   85                  90                  95
   Cys Gln His Ser Arg Glu Leu Pro Tyr Thr Phe Gly Gly Gly Thr Lys
               100                 105                 110
   Leu Glu Ile Lys Arg Gly Gly Gly Gly Ser Gly Gly Gly Gly Ser Gly
           115                 120                 125
   Gly Gly Gly Ser Gly Gly Gly Gly Ser Glu Val Gln Leu Val Glu Ser
       130                 135                 140
   Gly Gly Thr Leu Val Lys Pro Gly Gly Ser Leu Lys Leu Ser Cys Ala
   145                 150                 155                 160
   Ala Ser Gly Phe Thr Phe Asn Asn Tyr Pro Met Ser Trp Val Arg Gln
                   165                 170                 175
   Gly Pro Glu Lys Arg Leu Glu Trp Val Ala Thr Ile Ser Tyr Gly Gly
               180                 185                 190
   Asn Tyr Thr Phe Tyr Pro Asp Asn Leu Lys Gly Arg Phe Thr Ile Ser
           195                 200                 205
   Arg Asp Asn Ala Lys Asn Asn Leu Tyr Leu Gln Met Ser His Leu Lys
       210                 215                 220
   Ser Ala Asp Thr Ala Ile Tyr Tyr Cys Ala Arg His Phe Asp Tyr Asp
   225                 230                 235                 240
   Phe Phe Asp Phe Trp Gly Thr Gly Thr Thr Leu Thr Val Ser Ser
                   245                 250                 255
<210> 15
   <211> 753
   <212> DNA
   <213> artificial sequence
<220>
   <223> /note="Description of artificial sequence: CD123 scFv Klon 54"
<400> 15
   atggcggact acaaagacat tgtgatgaca cagtctcaca aattcatgtc cacatcagta      60
   ggagacaggg tcagcatcac ctgcaaggcc agtcaggatg tgagtactgc tgtagcctgg     120
   tatcaacaga aaccaggaca atctcctaaa ctactgattt actcggcatc ctaccggtac     180
   actggagtcc ctgatcgctt cactggcagt ggatctggga cggatttcac tttcaccatc     240
   agcagtgtgc aggctgaaga cctggcagtt tattactgtc agcaacatta tagtactccg     300
   tacacgttcg gaggggggac caagctggaa ataaaacgcg gtggtggtgg ttctggtggt     360
   ggtggttctg gcggcggcgg ctccggtggt ggtggatccg aggtgcagct agttgagtct     420
   gggggaacct tagtgaagcc tggagggtcc ctgaaactct cctgtgcagc ctctggattc     480
   actttcaata actatcccat gtcttgggtt cgccagggtc cggaaaagag gctggagtgg     540
   gtcgccacca ttagttatgg tggtaattac accttctatc cagacaatct aaagggccga     600
   ttcaccatct ccagagacaa tgccaagaac aacctgtacc tgcaaatgag ccatctgaag     660
   tctgcggaca cagccatata ttactgtgcc agacattttg attacgactt ctttgacttc     720
   tggggccaag gcaccagtct cacagtctcc tcg                                   753
<210> 16
   <211> 251
   <212> PRT
   <213> artificial sequence
<220>
   <223> /note="Description of artificial sequence: CD123 scFv Klon 54"
<400> 16
   Met Ala Asp Tyr Lys Asp Ile Val Met Thr Gln Ser His Lys Phe Met
   1               5                   10                  15
   Ser Thr Ser Val Gly Asp Arg Val Ser Ile Thr Cys Lys Ala Ser Gln
   20 25 30
   Asp Val Ser Thr Ala Val Ala Trp Tyr Gln Gln Lys Pro Gly Gln Ser
   35 40 45
   Pro Lys Leu Leu Ile Tyr Ser Ala Ser Tyr Arg Tyr Thr Gly Val Pro
   50 55 60
   Asp Arg Phe Thr Gly Ser Gly Ser Gly Thr Asp Phe Thr Phe Thr Ile
   65 70 75 80
   Ser Ser Val Gln Ala Glu Asp Leu Ala Val Tyr Tyr Cys Gln Gln His
   85 90 95
   Tyr Ser Thr Pro Tyr Thr Phe Gly Gly Gly Thr Lys Leu Glu Ile Lys
   100 105 110
   Arg Gly Gly Gly Gly Ser Gly Gly Gly Gly Ser Gly Gly Gly Gly Ser
   115 120 125
   Gly Gly Gly Gly Ser Glu Val Gln Leu Val Glu Ser Gly Gly Thr Leu
   130 135 140
   Val Lys Pro Gly Gly Ser Leu Lys Leu Ser Cys Ala Ala Ser Gly Phe
   145 150 155 160
   Thr Phe Asn Asn Tyr Pro Met Ser Trp Val Arg Gln Gly Pro Glu Lys
   165 170 175
   Arg Leu Glu Trp Val Ala Thr Ile Ser Tyr Gly Gly Asn Tyr Thr Phe
   180 185 190
   Tyr Pro Asp Asn Leu Lys Gly Arg Phe Thr Ile Ser Arg Asp Asn Ala
   195 200 205
   Lys Asn Asn Leu Tyr Leu Gln Met Ser His Leu Lys Ser Ala Asp Thr
   210 215 220
   Ala Ile Tyr Tyr Cys Ala Arg His Phe Asp Tyr Asp Phe Phe Asp Phe
   225 230 235 240
   Trp Gly Gln Gly Thr Ser Leu Thr Val Ser Ser
   245 250
<210> 17
   <211> 780
   <212> DNA
   <213> artificial sequence
<220>
   <223> /note="Description of artificial sequence: CD123 scFv Klon 60"
<400> 17
   atggcggact acaaagacat tctgatgacc cagtctcaaa aattcatgtc cacatcagta      60
   ggagacaggg tcagcgtcac ctgcaaggcc agtcagaatg tgggtactaa tgtagcctgg     120
   tatcaacaga aaccagggca atctcctaaa gcactgattt actcggcatt tgaccggtac     180
   agtggagtcc ctgatcgctt cacaggcagt ggatctggga cagatttcac tctcaccatc     240
   agcaatgtgc agtctgaaga cttggcagag tatttctgtc agcaatataa cagctatcct     300
   ctgacgttcg gtggaggcac caagctggaa ataaaacgcg gtggtggtgg ttctggtggt     360
   ggtggttctg gcggcggcgg ctccggtggt ggtggatccc aggtgcagct tgtagagact     420
   ggaggaggct tggtacagcc tgggggttct ctgagtctct cctgtgcagc ttctggattc    480
   accttcactg attactacat gagctgggtc cgccagcctc cagggaaggc acttgagtgg     540
   ttggctttga ttagaagcaa agctgatggt tacacaacag aatacagtgc atctgtgaag     600
   ggtcggttca ccctctccag agatgattcc caaagcatcc tctatcttca aatgaatgcc     660
   ctgagacctg aagacagtgc cacttattac tgtgcaagag atgcggccta ctatagttac     720
   tatagtcccg agggggctat ggactactgg ggtcaaggaa cctcagtcac cgtctcctcg      780
<210> 18
   <211> 260
   <212> PRT
   <213> artificial sequence
<220>
   <223> /note="Description of artificial sequence: CD123 scfv Klon 60"
<400> 18
   Met Ala Asp Tyr Lys Asp Ile Leu Met Thr Gln Ser Gln Lys Phe Met
   1               5                   10                  15
   Ser Thr Ser Val Gly Asp Arg Val Ser Val Thr Cys Lys Ala Ser Gln
               20                  25                  30
   Asn Val Gly Thr Asn Val Ala Trp Tyr Gln Gln Lys Pro Gly Gln Ser
           35                  40                  45
   Pro Lys Ala Leu Ile Tyr Ser Ala Phe Asp Arg Tyr Ser Gly Val Pro
       50                  55                  60
   Asp Arg Phe Thr Gly Ser Gly Ser Gly Thr Asp Phe Thr Leu Thr Ile
   65                  70                  75                  80
   Ser Asn Val Gln Ser Glu Asp Leu Ala Glu Tyr Phe Cys Gln Gln Tyr
                   85                  90                  95
   Asn Ser Tyr Pro Leu Thr Phe Gly Gly Gly Thr Lys Leu Glu Ile Lys
   100 105 110
   Arg Gly Gly Gly Gly Ser Gly Gly Gly Gly Ser Gly Gly Gly Gly Ser
   115 120 125
   Gly Gly Gly Gly Ser Gln Val Gln Leu Val Glu Thr Gly Gly Gly Leu
   130 135 140
   Val Gln Pro Gly Gly Ser Leu Ser Leu Ser Cys Ala Ala Ser Gly Phe
   145 150 155 160
   Thr Phe Thr Asp Tyr Tyr Met Ser Trp Val Arg Gln Pro Pro Gly Lys
   165 170 175
   Ala Leu Glu Trp Leu Ala Leu Ile Arg Ser Lys Ala Asp Gly Tyr Thr
   180 185 190
   Thr Glu Tyr Ser Ala Ser Val Lys Gly Arg Phe Thr Leu Ser Arg Asp
   195 200 205
   Asp Ser Gln Ser Ile Leu Tyr Leu Gln Met Asn Ala Leu Arg Pro Glu
   210 215 220
   Asp Ser Ala Thr Tyr Tyr Cys Ala Arg Asp Ala Ala Tyr Tyr Ser Tyr
   225 230 235 240
   Tyr Ser Pro Glu Gly Ala Met Asp Tyr Trp Gly Gln Gly Thr Ser Val
   245 250 255
   Thr Val Ser Ser
   260
<210> 19
   <211> 771
   <212> DNA
   <213> artificial sequence
<220>
   <223> /note="Description of artificial sequence: CD123 scFv Klon 74"
<400> 19
   atggcggact acaaagatgt ccagatgacc cagtctccat cctccctagc tgtgtcagtt 60
   ggagagaagg ttactatgag ctgcaagtcc agtcagagcc ttttctttag tagcaatcaa 120
   aagaactact tggcctggta ccagcagaaa ccagggcagt ctcctaaact gctgatttcc 180
   tgggcatcca ctagggaatc tggggtccct gatcgcttca caggcagtgg atctgggaca 240
   gatttcactc tcaccatcag cagtgtgaag gctgaagacc tggcagttta ttactgtcag 300
   caattttata gctttcctcg gacgttcggt ggaggcacca agctggaaat aaaacgcggt 360
   ggtggtggtt ctggtggtgg tggttctggc ggcggcggct ccggtggtgg tggatccgaa 420
   gtgaagctgg tggagtctgg gggaggctta gtgaagcctg gagggtccct gaaactctcc 480
   tgtgcagcct ctggattcac tttcagtagc tatgccatgt cttgggttcg ccagactccg 540
   gaaaagaggc tggagtgggt cgcaaccgtt agttatggtg gtagttacac cttctatcca 600
   gacaatgtaa agggccgatt caccatctcc agagacaatg ccaagaacaa cctgtacctg 660
   caaatgagcc atctgaagtc tgaggacaca gccatgtatt attgtgcaag agattatgat 720
   tacgacttct ttgactcctg gggccaaggc accactctca cagtctcctc g 771
<210> 20
   <211> 257
   <212> PRT
   <213> artificial sequence
<220>
   <223> /note="Description of artificial sequence: CD123 scFv Klon 74"
<400> 20
   Met Ala Asp Tyr Lys Asp Val Gln Met Thr Gln Ser Pro Ser Ser Leu
   1               5                   10                  15
   Ala Val Ser Val Gly Glu Lys Val Thr Met Ser Cys Lys Ser Ser Gln
               20                  25                  30
   Ser Leu Phe Phe Ser Ser Asn Gln Lys Asn Tyr Leu Ala Trp Tyr Gln
           35                  40                  45
   Gln Lys Pro Gly Gln Ser Pro Lys Leu Leu Ile Ser Trp Ala Ser Thr
       50                  55                  60
   Arg Glu Ser Gly Val Pro Asp Arg Phe Thr Gly Ser Gly Ser Gly Thr
   65                  70                  75                  80
   Asp Phe Thr Leu Thr Ile Ser Ser Val Lys Ala Glu Asp Leu Ala Val
                   85                  90                  95
   Tyr Tyr Cys Gln Gln Phe Tyr Ser Phe Pro Arg Thr Phe Gly Gly Gly
               100                 105                 110
   Thr Lys Leu Glu Ile Lys Arg Gly Gly Gly Gly Ser Gly Gly Gly Gly
           115                 120                 125
   Ser Gly Gly Gly Gly Ser Gly Gly Gly Gly Ser Glu Val Lys Leu Val
       130                 135                 140
   Glu Ser Gly Gly Gly Leu Val Lys Pro Gly Gly Ser Leu Lys Leu Ser
   145                 150                 155                 160
   Cys Ala Ala Ser Gly Phe Thr Phe Ser Ser Tyr Ala Met Ser Trp Val
                   165                 170                 175
   Arg Gln Thr Pro Glu Lys Arg Leu Glu Trp Val Ala Thr Val Ser Tyr
               180                 185                 190
   Gly Gly Ser Tyr Thr Phe Tyr Pro Asp Asn Val Lys Gly Arg Phe Thr
           195                 200                 205
   Ile Ser Arg Asp Asn Ala Lys Asn Asn Leu Tyr Leu Gln Met Ser His
       210                 215                 220
   Leu Lys Ser Glu Asp Thr Ala Met Tyr Tyr Cys Ala Arg Asp Tyr Asp
   225                 230                 235                 240
   Tyr Asp Phe Phe Asp Ser Trp Gly Gln Gly Thr Thr Leu Thr Val Ser
                   245                 250                 255
   Ser
<210> 21
   <211> 753
   <212> DNA
   <213> artificial sequence
<220>
   <223> /note="Description of artificial sequence: CD123 scFv Klon 101"
<400> 21
   atggcggact acaaagacat tgtgatgaca cagtctcaca aattcatgtc cacatcagta    60
   ggagacaggg tcatcatcac ctgcaaggcc agtcaggatg tgggttctgc tgtagcctgg   120
   tatcaacaga aaccaggaca atctcctaaa ctactgattt actgggcatc cacccggcac   180
   actggagtcc ctgatcgctt cacaggcagt ggatctggga cagatttcac tctcaccatt   240
   agcaatgtgc agtctgaaga cttggcagat tatttctgtc agcaatatag cagctatcct   300
   cggacgttcg gtggaggcac caagctggaa atcaaacgcg gtggtggtgg ttctggtggt   360
   ggtggttctg gcggcggcgg ctccggtggt ggtggatccg aggtgcagct agttgagtct   420
   gggggaggct tagtgaagcc tggagggtcc ctgaaactct cctgtgcagc ctctggattc   480
   actttcagta gctatgccat gtcttgggtt cgccagactc cggaaaagag gctggagtgg   540
   gtcgcaaccg ttagttatgg tggtagttac accttctatc cagacaatgt aaagggccga   600
   ttcaccatct ccagagacaa tgccaagaac aacctgtacc tgcaaatgag ccatctgaag   660
   tctgaggaca cagccatgta ttattgtgca agagattatg attacgactt ctttgactcc   720
   tggggccaag gcaccacggt caccgtctcc tcg                                753
<210> 22
   <211> 251
   <212> PRT
   <213> artificial sequence
<220>
   <223> /note="Description of artificial sequence: CD123 scFv Klon 101"
<400> 22
   Met Ala Asp Tyr Lys Asp Ile Val Met Thr Gln Ser His Lys Phe Met
   1               5                   10                  15
   Ser Thr Ser Val Gly Asp Arg Val Ile Ile Thr Cys Lys Ala Ser Gln
               20                  25                  30
   Asp Val Gly Ser Ala Val Ala Trp Tyr Gln Gln Lys Pro Gly Gln Ser
           35                  40                  45
   Pro Lys Leu Leu Ile Tyr Trp Ala Ser Thr Arg His Thr Gly Val Pro
       50                  55                  60
   Asp Arg Phe Thr Gly Ser Gly Ser Gly Thr Asp Phe Thr Leu Thr Ile
   65                  70                  75                  80
   Ser Asn Val Gln Ser Glu Asp Leu Ala Asp Tyr Phe Cys Gln Gln Tyr
                   85                  90                  95
   Ser Ser Tyr Pro Arg Thr Phe Gly Gly Gly Thr Lys Leu Glu Ile Lys
               100                 105                 110
   Arg Gly Gly Gly Gly Ser Gly Gly Gly Gly Ser Gly Gly Gly Gly Ser
           115                 120                 125
   Gly Gly Gly Gly Ser Glu Val Gln Leu Val Glu Ser Gly Gly Gly Leu
       130                 135                 140
   Val Lys Pro Gly Gly Ser Leu Lys Leu Ser Cys Ala Ala Ser Gly Phe
   145                 150                 155                 160
   Thr Phe Ser Ser Tyr Ala Met Ser Trp Val Arg Gln Thr Pro Glu Lys
                   165                 170                 175
   Arg Leu Glu Trp Val Ala Thr Val Ser Tyr Gly Gly Ser Tyr Thr Phe
               180                 185                 190
   Tyr Pro Asp Asn Val Lys Gly Arg Phe Thr Ile Ser Arg Asp Asn Ala
           195                 200                 205
   Lys Asn Asn Leu Tyr Leu Gln Met Ser His Leu Lys Ser Glu Asp Thr
       210                 215                 220
   Ala Met Tyr Tyr Cys Ala Arg Asp Tyr Asp Tyr Asp Phe Phe Asp Ser
   225 230 235 240
   Trp Gly Gln Gly Thr Thr Val Thr Val Ser Ser
   245 250
<210> 23
   <211> 771
   <212> DNA
   <213> artificial sequence
<220>
   <223> /note="Description of artificial sequence: CD123 scFv Klon 120"
<400> 23
   atggcggact acaaagacat tgtgatgaca cagtctccat cctccctagc tgtgtcagtt 60
   ggagagaagg ttactatgag ctgcaagtcc agtcagagcc ttttatttag tagtaatcaa 120
   aagaactatt tggcctggta tcaacagaaa ccagggcagt ctcctaaact gctgatttac 180
   tgggcatcca ctagggaatc tggggtccct gatcgcttca caggcagtgg atctgggaca 240
   gatttcactc tcaccatcag cagtgtgaag gctgaagacc tggcagttta ttactgtcag 300
   caattttata gctttcctcg gacgttcggt ggaggcacca agctggaaat aaaacgcggt 360
   ggtggtggtt ctggtggtgg tggttctggc ggcggcggct ccggtggtgg tggatccgag 420
   gtgcagctag ttgagtctgg gggaggctta gtgaagcctg gagggtccct gaaactctcc 480
   tgtgcagcct ctggattcac tttcagtagc tatgccatgt cttgggttcg ccagactccg 540
   gaaaagaggc tggagtgggt cgcaaccgtt agttatggtg gtagttacac cttctatcca 600
   gacaatgtaa agggccgatt caccatctcc agagacaatg ccaagaacaa cctgtacctg 660
   caaatgagcc atctgaagtc tgaggacaca gccatgtatt attgtgcaag agattatgat 720
   tacgacttct ttgactcctg gggccaaggc accacggtca cagtctcctc g 771
<210> 24
   <211> 257
   <212> PRT
   <213> artificial sequence
<220>
   <223> /note="Description of artificial sequence: CD123 scFv Klon 120"
<400> 24
   Met Ala Asp Tyr Lys Asp Ile Val Met Thr Gln Ser Pro Ser Ser Leu
   1 5 10 15
   Ala Val Ser Val Gly Glu Lys Val Thr Met Ser Cys Lys Ser Ser Gln
   20 25 30
   Ser Leu Leu Phe Ser Ser Asn Gln Lys Asn Tyr Leu Ala Trp Tyr Gln
   35 40 45
   Gln Lys Pro Gly Gln Ser Pro Lys Leu Leu Ile Tyr Trp Ala Ser Thr
   50 55 60
   Arg Glu Ser Gly Val Pro Asp Arg Phe Thr Gly Ser Gly Ser Gly Thr
   65 70 75 80
   Asp Phe Thr Leu Thr Ile Ser Ser Val Lys Ala Glu Asp Leu Ala Val
   85 90 95
   Tyr Tyr Cys Gln Gln Phe Tyr Ser Phe Pro Arg Thr Phe Gly Gly Gly
   100 105 110
   Thr Lys Leu Glu Ile Lys Arg Gly Gly Gly Gly Ser Gly Gly Gly Gly
   115 120 125
   Ser Gly Gly Gly Gly Ser Gly Gly Gly Gly Ser Glu Val Gln Leu Val
   130 135 140
   Glu Ser Gly Gly Gly Leu Val Lys Pro Gly Gly Ser Leu Lys Leu Ser
   145 150 155 160
   Cys Ala Ala Ser Gly Phe Thr Phe Ser Ser Tyr Ala Met Ser Trp Val
   165 170 175
   Arg Gln Thr Pro Glu Lys Arg Leu Glu Trp Val Ala Thr Val Ser Tyr
   180 185 190
   Gly Gly Ser Tyr Thr Phe Tyr Pro Asp Asn Val Lys Gly Arg Phe Thr
   195 200 205
   Ile Ser Arg Asp Asn Ala Lys Asn Asn Leu Tyr Leu Gln Met Ser His
   210 215 220
   Leu Lys Ser Glu Asp Thr Ala Met Tyr Tyr Cys Ala Arg Asp Tyr Asp
   225 230 235 240
   Tyr Asp Phe Phe Asp Ser Trp Gly Gln Gly Thr Thr Val Thr Val Ser
   245 250 255
   Ser
<210> 25
   <211> 753
   <212> DNA
   <213> artificial sequence
<220>
   <223> /note="Description of artificial sequence: CD123 scFv Klon 148"
<400> 25
   atggcggact acaaagatgt tgtgatgacc cagtctcaaa aattcatgtc cacatcagta     60
   ggagacaggg tcagcgtcac ctgcaaggcc agtcagaatg tgggtactaa tgtagcctgg    120
   tatcaacaga aaccagggca atctcctaaa gcactgattt actcggcatc ctaccggtac    180
   agtggagtcc ctgatcgctt cacaggcagt ggatctggga cagatttcac tctcaccatc    240
   agcaatgtgc agtctgaaga cttggcagag tatttctgtc agcaatataa cagctatccg    300
   tacacgttcg gaggggggac caagctggaa atcaaacgcg gtggtggtgg ttctggtggt    360
   ggtggttctg gcggcggcgg ctccggtggt ggtggatccg aggtgcagct agttgagtct    420
   gggggaacct tagtgaagcc tggagggtcc ctgaaactct cctgtgcagc ctctggattc    480
   actttcaata actatcccat gtcttgggtt cgccagggtc cggaaaagag gctggagtgg    540
   gtcgccacca ttagttatgg tggtaattac accttctatc cagacaatct aaagggccga    600
   ttcaccatct ccagagacaa tgccaagaac aacctgtacc tgcaaatgag ccatctgaag    660
   tctgcggaca cagccatata ttactgtgcc agacattttg attacgactt ctttgacttc    720
   tggggccaag gcaccactct cacagtctcc tcg                                 753
<210> 26
   <211> 251
   <212> PRT
   <213> artificial sequence
<220>
   <223> /note="Description of artificial sequence: CD123 scFv klon 148"
<400> 26
   Met Ala Asp Tyr Lys Asp Val Val Met Thr Gln Ser Gln Lys Phe Met
   1               5                   10                  15
   Ser Thr Ser Val Gly Asp Arg Val Ser Val Thr Cys Lys Ala Ser Gln
               20                  25                  30
   Asn Val Gly Thr Asn Val Ala Trp Tyr Gln Gln Lys Pro Gly Gln Ser
           35                  40                  45
   Pro Lys Ala Leu Ile Tyr Ser Ala Ser Tyr Arg Tyr Ser Gly Val Pro
       50                  55                  60
   Asp Arg Phe Thr Gly Ser Gly Ser Gly Thr Asp Phe Thr Leu Thr Ile
   65                  70                  75                  80
   Ser Asn Val Gln Ser Glu Asp Leu Ala Glu Tyr Phe Cys Gln Gln Tyr
                   85                  90                  95
   Asn Ser Tyr Pro Tyr Thr Phe Gly Gly Gly Thr Lys Leu Glu Ile Lys
               100                 105                 110
   Arg Gly Gly Gly Gly Ser Gly Gly Gly Gly Ser Gly Gly Gly Gly Ser
           115                 120                 125
   Gly Gly Gly Gly Ser Glu Val Gln Leu Val Glu Ser Gly Gly Thr Leu
       130                 135                 140
   Val Lys Pro Gly Gly Ser Leu Lys Leu Ser Cys Ala Ala Ser Gly Phe
   145                 150                 155                 160
   Thr Phe Asn Asn Tyr Pro Met Ser Trp Val Arg Gln Gly Pro Glu Lys
                   165                 170                 175
   Arg Leu Glu Trp Val Ala Thr Ile Ser Tyr Gly Gly Asn Tyr Thr Phe
               180                 185                 190
   Tyr Pro Asp Asn Leu Lys Gly Arg Phe Thr Ile Ser Arg Asp Asn Ala
           195                 200                 205
   Lys Asn Asn Leu Tyr Leu Gln Met Ser His Leu Lys Ser Ala Asp Thr
       210                 215                 220
   Ala Ile Tyr Tyr Cys Ala Arg His Phe Asp Tyr Asp Phe Phe Asp Phe
   225                 230                 235                 240
   Trp Gly Gln Gly Thr Thr Leu Thr Val Ser Ser
                   245                 250
<210> 27
   <211> 2436
   <212> DNA
   <213> artificial sequence
<220>
   <223> /note="Description of artificial sequence: Sctb [123 x ds16 x 33]
<400> 27
   atggcggact acaaagatat tgtgatgacc cagtctcaca aattcatgtc cacatcagta     60
   ggagacaggg tcaacatcac ctgcaaggcc agtcagaatg tggatagtgc tgtagcctgg    120
   tatcaacaga aaccagggca atctcctaaa gcactgattt actcggcatc ctaccggtac    180
   agtggagtcc ctgatcgctt cacaggcagg ggatctggga cagatttcac tctcaccatc    240
   agcagtgtgc aggctgaaga cctggcagtt tattactgtc agcaatatta tagcactccg    300
   tggacgttcg gtggaggcac caagctggaa atcaaacgtg gtggtggtgg ttctggtggt    360
   ggtggttctg gcggcggcgg ctccggtggt ggtggatccg aggtgaagct ggtggagtct    420
   ggaggaggct tggtacagcc tgggggttct ctgagtctct cctgtgcagc ttctggattc    480
   accttcactg attactacat gagctgggtc cgccagcctc cagggaaggc acttgagtgg 540
   ttggctttga ttagaagcaa agctgatggt tacacaacag aatacagtgc atctgtgaag 600
   ggtcggttca ccctctccag agatgattcc caaagcatcc tctatcttca aatgaatgcc 660
   ctgagacctg aagacagtgc cacttattac tgtgcaagag atgcggccta ctatagttac 720
   tatagtcccg agggggctat ggactactgg ggtcaaggaa cctcagtcac cgtctcctcg 780
   gcctcggggg ccggtggtgg cggcagtggt ggtggcggca gtggtggtgg cggcagtggt 840
   ggtggcggca gtgcggccgc ggactacaaa gacattgtgc tgacccaatc tccagcttct 900
   ttggctgtgt ctctagggca gagggccacc atctcctgca aggccagcca aagtgttgat 960
   tttgatggtg atagttttat gaactggtac caacagaaac caggacagcc acccaaactc 1020
   ctcatctata ctacatccaa tctagaatct gggatcccag ccaggtttag tgccagtggg 1080
   tctgggacag acttcaccct caacatccat cctgtggagg aggaggatac tgcaacctat 1140
   tactgtcagc aaagtaatga ggatccgtac acgttcggat gcgggaccaa gctggagctg 1200
   aaacgtggtg gtggtggttc tggtggtggt ggttctggcg gcggcggctc cggtggtggt 1260
   ggatcccagg ttactctgaa agagtctggc cctgggatat tgcagccctc ccagaccctc 1320
   agtctgactt gttctttctc tgggttttca ctgaggactt ctggtatggg tgtaggctgg 1380
   attcgtcagc cttcagggaa gtgcctagag tggctggcac acatttggtg ggatgatgac 1440
   aagcgctata atccagccct gaagagccga ctgacaatct ccaaggatac ctccagcaac 1500
   caggtattcc tcaaaatcgc cagtgtggac actgcagata ctgccacata ctactgtgct 1560
   caaataaacc ccgcctggtt tgcttactgg ggccaaggga ctctggtcac tgtctctcga 1620
   ggtggtggtg gttctggagg aggaggatct ggcggcggcg gctctggagg cggaggctct 1680
   atggcggact acaaagacat tgtgatgacc cagtctcaca aattcctgct tgtatcagta 1740
   ggagacaggg tcagcatcac ctgcaaggcc agtcaggatg tgagtactgc tgtagcctgg 1800
   tatcaacaga aaccaggaca atctcctaaa ctactgattt actcggcatc ctaccggtac 1860
   actggagtcc ctgatcgctt cataggcagt ggatctggga cagatttcac tcttaccatc 1920
   agcagtgtgc aggctgaaga cctggcagat tacttctgtc agcaacatta tagcactccg 1980
   ctcacgttcg gtgctgggac caagctggaa ataaaacgtg gtggtggtgg ttctggtggt 2040
   ggtggttctg gcggcggcgg ctccagtggt ggtggatccg aggtgcagct gaaggagtca 2100
   ggacctggcc tggtggcgcc ctcacagagc ctgtccatca catgcactgt ctcagggttc 2160
   ccattaacca gctatggtgt aagctgggtt cgccagcctc caggaaaggg tctggagtgg 2220
   ctgggagtaa tatggggtga cgggagcaca aattatcatt cagctctcat atccagactg 2280
   agcatcagca aggataactc caagagccaa gttttcttaa aactgaacaa tctgcaaact 2340
   gatgacacag ccacgtacta ctgtgcccgt gatacttact acccctacta tgctatggac 2400
   tattggggtc aaggaacctc agtcaccgtc tcctcg 2436
<210> 28
   <211> 812
   <212> PRT
   <213> artificial sequence
<220>
   <223> /note="Description of artificial sequence: Sctb [123 x ds16 x 33]"
<400> 28
   Met Ala Asp Tyr Lys Asp Ile Val Met Thr Gln Ser His Lys Phe Met
   1               5                   10                  15
   Ser Thr Ser Val Gly Asp Arg Val Asn Ile Thr Cys Lys Ala Ser Gln
               20                  25                  30
   Asn Val Asp Ser Ala Val Ala Trp Tyr Gln Gln Lys Pro Gly Gln Ser
           35                  40                  45
   Pro Lys Ala Leu Ile Tyr Ser Ala Ser Tyr Arg Tyr Ser Gly Val Pro
       50                  55                  60
   Asp Arg Phe Thr Gly Arg Gly Ser Gly Thr Asp Phe Thr Leu Thr Ile
   65                  70                  75                  80
   Ser Ser Val Gln Ala Glu Asp Leu Ala Val Tyr Tyr Cys Gln Gln Tyr
                   85                  90                  95
   Tyr Ser Thr Pro Trp Thr Phe Gly Gly Gly Thr Lys Leu Glu Ile Lys
               100                 105                 110
   Arg Gly Gly Gly Gly Ser Gly Gly Gly Gly Ser Gly Gly Gly Gly Ser
           115                 120                 125
   Gly Gly Gly Gly Ser Glu Val Lys Leu Val Glu Ser Gly Gly Gly Leu
       130                 135                 140
   Val Gln Pro Gly Gly Ser Leu Ser Leu Ser Cys Ala Ala Ser Gly Phe
   145                 150                 155                 160
   Thr Phe Thr Asp Tyr Tyr Met Ser Trp Val Arg Gln Pro Pro Gly Lys
                   165                 170                 175
   Ala Leu Glu Trp Leu Ala Leu Ile Arg Ser Lys Ala Asp Gly Tyr Thr
               180                 185                 190
   Thr Glu Tyr Ser Ala Ser Val Lys Gly Arg Phe Thr Leu Ser Arg Asp
           195                 200                 205
   Asp Ser Gln Ser Ile Leu Tyr Leu Gln Met Asn Ala Leu Arg Pro Glu
       210                 215                 220
   Asp Ser Ala Thr Tyr Tyr Cys Ala Arg Asp Ala Ala Tyr Tyr Ser Tyr
   225                 230                 235                 240
   Tyr Ser Pro Glu Gly Ala Met Asp Tyr Trp Gly Gln Gly Thr Ser Val
                   245                 250                 255
   Thr Val Ser Ser Ala Ser Gly Ala Gly Gly Gly Gly Ser Gly Gly Gly
               260                 265                 270
   Gly Ser Gly Gly Gly Gly Ser Gly Gly Gly Gly Ser Ala Ala Ala Asp
           275                 280                 285
   Tyr Lys Asp Ile Val Leu Thr Gln Ser Pro Ala Ser Leu Ala Val Ser
       290                 295                 300
   Leu Gly Gln Arg Ala Thr Ile Ser Cys Lys Ala Ser Gln Ser Val Asp
   305                 310                 315                 320
   Phe Asp Gly Asp Ser Phe Met Asn Trp Tyr Gln Gln Lys Pro Gly Gln
                   325                 330                 335
   Pro Pro Lys Leu Leu Ile Tyr Thr Thr Ser Asn Leu Glu Ser Gly Ile
               340                 345                 350
   Pro Ala Arg Phe Ser Ala Ser Gly Ser Gly Thr Asp Phe Thr Leu Asn
           355                 360                 365
   Ile His Pro Val Glu Glu Glu Asp Thr Ala Thr Tyr Tyr Cys Gln Gln
       370                 375                 380
   Ser Asn Glu Asp Pro Tyr Thr Phe Gly Cys Gly Thr Lys Leu Glu Leu
   385                 390                 395                 400
   Lys Arg Gly Gly Gly Gly Ser Gly Gly Gly Gly Ser Gly Gly Gly Gly
                   405                 410                 415
   Ser Gly Gly Gly Gly Ser Gln Val Thr Leu Lys Glu Ser Gly Pro Gly
               420                 425                 430
   Ile Leu Gln Pro Ser Gln Thr Leu Ser Leu Thr Cys Ser Phe Ser Gly
           435                 440                 445
   Phe Ser Leu Arg Thr Ser Gly Met Gly Val Gly Trp Ile Arg Gln Pro
       450                 455                 460
   Ser Gly Lys Cys Leu Glu Trp Leu Ala His Ile Trp Trp Asp Asp Asp
   465 470 475 480
   Lys Arg Tyr Asn Pro Ala Leu Lys Ser Arg Leu Thr Ile Ser Lys Asp
   485 490 495
   Thr Ser Ser Asn Gln Val Phe Leu Lys Ile Ala Ser Val Asp Thr Ala
   500 505 510
   Asp Thr Ala Thr Tyr Tyr Cys Ala Gln Ile Asn Pro Ala Trp Phe Ala
   515 520 525
   Tyr Trp Gly Gln Gly Thr Leu Val Thr Val Ser Arg Gly Gly Gly Gly
   530 535 540
   Ser Gly Gly Gly Gly Ser Gly Gly Gly Gly Ser Gly Gly Gly Gly Ser
   545 550 555 560
   Met Ala Asp Tyr Lys Asp Ile Val Met Thr Gln Ser His Lys Phe Leu
   565 570 575
   Leu Val Ser Val Gly Asp Arg Val Ser Ile Thr Cys Lys Ala Ser Gln
   580 585 590
   Asp Val Ser Thr Ala Val Ala Trp Tyr Gln Gln Lys Pro Gly Gln Ser
   595 600 605
   Pro Lys Leu Leu Ile Tyr Ser Ala Ser Tyr Arg Tyr Thr Gly Val Pro
   610 615 620
   Asp Arg Phe Ile Gly Ser Gly Ser Gly Thr Asp Phe Thr Leu Thr Ile
   625 630 635 640
   Ser Ser Val Gln Ala Glu Asp Leu Ala Asp Tyr Phe Cys Gln Gln His
   645 650 655
   Tyr Ser Thr Pro Leu Thr Phe Gly Ala Gly Thr Lys Leu Glu Ile Lys
   660 665 670
   Arg Gly Gly Gly Gly Ser Gly Gly Gly Gly Ser Gly Gly Gly Gly Ser
   675 680 685
   Ser Gly Gly Gly Ser Glu Val Gln Leu Lys Glu Ser Gly Pro Gly Leu
   690 695 700
   Val Ala Pro Ser Gln Ser Leu Ser Ile Thr Cys Thr Val Ser Gly Phe
   705 710 715 720
   Pro Leu Thr Ser Tyr Gly Val Ser Trp Val Arg Gln Pro Pro Gly Lys
   725 730 735
   Gly Leu Glu Trp Leu Gly Val Ile Trp Gly Asp Gly Ser Thr Asn Tyr
   740 745 750
   His Ser Ala Leu Ile Ser Arg Leu Ser Ile Ser Lys Asp Asn Ser Lys
   755 760 765
   Ser Gln Val Phe Leu Lys Leu Asn Asn Leu Gln Thr Asp Asp Thr Ala
   770 775 780
   Thr Tyr Tyr Cys Ala Arg Asp Thr Tyr Tyr Pro Tyr Tyr Ala Met Asp
   785 790 795 800
   Tyr Trp Gly Gln Gly Thr Ser Val Thr Val Ser Ser
   805 810